# EUROPEAN PATENT SPECIFICATION

(11) **EP 2 181 589 B1**
(45) Date of publication and mention of the grant of the patent: **16.08.2017**
(21) Application number: 08253574.1
(22) Date of filing: 31.10.2008
(51) Int. Cl.: A01K 67/027, C07J 9/00, C07J 17/00

(54) **Rodent model of neurodegenerative disease administered with neurotoxic stigmasterol glucoside**
Mit neurotoxischem Stigmasterolglucosid verabreichtes Nagetiermodell von neurodegenerativer Erkrankung
Modèle rongeur de maladie dégénérative par administration de glucoside de stigmasterol neurotoxique

(43) Date of publication of application: 05.05.2010
(73) Proprietor: Shaw, Christopher Ariel, North Vancouver (CA); Neurodyn Life Sciences Inc., Vancouver, British Columbia, V6B 2E6 (CA)
(72) Inventor: SHAW, Christopher Ariel, North Vancouver (CA); KAY, Denis, Straford PE C1B 1Y8 (CA)
(74) Representative: Elsy, David

(56) References cited:
- WO-A-02/37122
- US-A1- 2006 252 705
- US-A1- 2011 280 805
- WILSON J M B ET AL: "BEHAVIORAL AND NEUROLOGICAL CORRELATES OF ALS-PARKINSONISM DEMENTIA COMPLEX IN ADULT MICE FED WASHED CYCAD FLOUR" NEUROMOLECULAR MEDICINE, HUMANA PRESS, US, vol. 3, no. 1, 1 January 2002 (2002-01-01), pages 207-221, XP008031202 ISSN: 1535-1084
- KHABAZIAN I, ET AL.: "Isolation of various forms of sterol beta-D-glucoside from the seed of Cycas circinalis: neurotoxicity and implications for ALS-parkinsonism dementia complex" J NEUROCHEM., vol. 82, no. 3, August 2002 (2002-08), pages 516-528, XP002521265
- WILSON ET AL: "Late appearance of glutamate transporter defects in a murine model of ALS-parkinsonism dementia complex" NEUROCHEMISTRY INTERNATIONAL, PERGAMON PRESS, OXFORD, GB, vol. 50, no. 7-8, 1 June 2007 (2007-06-01), pages 1067-1077, XP022129965 ISSN: 0197-0186
- LY PT, SINGH S, SHAW CA.: "Novel environmental toxins: steryl glycosides as a potential etiological factor for age-related neurodegenerative diseases." J NEUROSCI RES., vol. 85, no. 2, 5 December 2006 (2006-12-05), - 1 February 2007 (2007-02-01) pages 231-237, XP002521266
- SCHULZ ET AL: "Cycad toxins, Helicobacter pylori and parkinsonism: Cholesterol glucosides as the common denomenator" MEDICAL HYPOTHESES, EDEN PRESS, PENRITH, US, vol. 66, no. 6, 1 January 2006 (2006-01-01), pages 1222-1226, XP005347213 ISSN: 0306-9877
- WILSON J, SHAW CA.: "Commentary on: Return of the cycad hypothesis--does the amyotrophic lateral sclerosis/parkinsonism dementia complex (ALS/PDC) of Guam have new implications for global health?" NEUROPATHOL APPL NEUROBIOL, vol. 32, no. 3, June 2006 (2006-06), pages 341-343, XP002521268
- TABATA RC, WILSON JM, LY P, ZWIEGERS P, KWOK D, VAN KAMPEN JM, CASHMAN N, SHAW CA.: "Chronic exposure to dietary sterol glucosides is neurotoxic to motor neurons and induces an ALS-PDC phenotype." NEUROMOLECULAR MED., vol. 10, no. 1, 15 January 2008 (2008-01-15), pages 24-39, XP002521267
- BATTA ET AL: "Stigmasterol reduces plasma cholesterol levels and inhibits hepatic synthesis and intestinal absorption in the rat" METABOLISM, CLINICAL AND EXPERIMENTAL, W.B. SAUNDERS CO., PHILADELPHIA, PA, US, vol. 55, no. 3, 1 March 2006 (2006-03-01), pages 292-299, XP005285591 ISSN: 0026-0495
- KULKARNI S K ET AL: "Withania somnifera: An Indian ginseng" PROGRESS IN NEURO-PSYCHOPHARMACOLOGY & BIOLOGICAL PSYCHIATRY, OXFORD, GB, vol. 32, no. 5, 1 July 2008 (2008-07-01), pages 1093-1105, XP022734553 ISSN: 0278-5846 [retrieved on 2008-06-16]
- MISRA ET AL: "Withanolides from Withania somnifera roots" PHYTOCHEMISTRY, PERGAMON PRESS, GB, vol. 69, no. 4, 3 December 2007 (2007-12-03), pages 1000-1004, XP022473183 ISSN: 0031-9422
- "Table of Content" In: SAHGAL ARJUN .: "BEHAVIOURAL NEUROSCIENCE Volume I A Practical approach. Appendix A A", 1993, Oxford University press USA pages VIII-XV,

## Description

### TECHNICAL FIELD

The invention relates to animal models of neurodegenerative disease and methods using thereof. More particularly, the invention relates to the use of neurotoxic synthetic and/or purified stigmasterol (SG) glycoside in rodent models of neurodegenerative disease.

### BACKGROUND AND SUMMARY

The neurodegenerative diseases, Alzheimer's disease (AD), Parkinson's disease (PD), amyotrophic lateral sclerosis (ALS), and amyotrophic lateral sclerosis-parkinsonism dementia complex (ALS-PDC) are typically diagnosed only after behavioral deficits can be detected clinically (Arasaki and Tamaki, 1998; Leenders et al., 1990; Whitehouse et al., 1982). In Alzheimer's disease, neurons are lost from various regions of the cerebral cortex and hippocampus and manifest as the loss of cognitive functions (Odle, 2003). Parkinson's disease involves the degeneration of select portions of the nigral striatal system (Schapira and Olanow, 2003). Initially, terminal projections of dopamine-containing neurons are lost. Eventually, the cell bodies of the dopaminergic neurons are lost from the substantia nigra (SN). This outcome results in disturbance of motor control and induces tremor, hypokinesia and rigidity (Dauer and Przedborski, 2003). Amyotrophic lateral sclerosis at endstage is characterized by the progressive loss of spinal and cortical motor neurons controlling motor function, particularly the diaphragm (Rowland and Shneider, 2001), resulting in paralysis and death.

In each of the aforementioned diseases, relatively specific neuronal populations degenerate and result in particular behavioral outcomes. It is believed that AD, PD, and ALS are distinct diseases, arising from distinct etiologies and resulting in mutually exclusive behavioral and neuropathological outcomes. This view is based on differential primary symptoms and pathological findings. However, more recent work has pointed to considerable cross-over between the neurodegenerative diseases (see Calne and Eisen, 1989; Muchowski and Wacker, 2005). For example, both PD and ALS patients can exhibit a decline in cognitive function which is conventionally an AD attribute (Aarsland et al., 2003; Vaphiades et al., 2002). Also, AD patients can experience tremors, which is a feature primarily associated with PD (Yokoyama et al., 2002). Another overlap between AD and PD is that the gait disturbances experienced by some PD patients are a predictor for developing AD-like dementia (Verghese et al., 2002). ALS and PD have been shown to overlap significantly, and ALS cases with dementia and/or parkinsonism features are accordingly called ALS-plus (Zoccolella et al., 2002).

Assessments of post-mortem AD, PD, and ALS tissue have also uncovered many similarities between these disease states. In particular, the similarities in the molecular mechanisms associated with protein folding and aggregation have been gaining increasing attention (Muchowski and Wacker, 2005). Although the specific proteins that aggregate in particular neurodegenerative diseases are often unrelated in primary amino acid sequence, the characteristic lesions of AD, PD, and ALS typically contain fibrillar, amyloid-like structures with similar biochemical features (Dobson, 2003). However, instances of proteinacious inclusions possessing a common primary amino acid sequence have been observed across disease classifications. For example, neurofibrillary tangles (NFT) which are primarily associated with AD have been identified in some ALS (Kokubo et al., 2000) and PD (Arima et al., 1999) patients. On the same note, α-synuclein, first identified in the amyloid plaques of AD patients, has also been identified in PD Lewy bodies and Lewy neurites (Lucking and Brice, 2000).

Although the age-related diseases, AD, PD and ALS have similarities, the similarities are limited. In contrast, the neurological disease, amyotrophic lateral sclerosis-parkinsonism dementia complex (ALS-PDC) is characterized by a wide range of behavioral and neuropathological attributes shared with each of the age-related diseases and many other neurological diseases. ALS-PDC can express as the classical form of ALS or as a form of parkinsonism with AD features. Moreover, some ALS-PDC patients of Guam or Rota have been observed to present with a combination of these symptoms (Steele and Guzman, 1987). Accordingly, it is believed that understanding ALS-PDC will result in a better understanding of neurological diseases as a whole.

In order to better understand the underlying neurological mechanisms of these disease states at both a cellular level and at the biochemical level, as well as, at the level of the whole animal, animal models of neurodegenerative diseases are needed. Ideally, the animals should display symptoms that approximate the symptoms displayed by humans suffering from these neurodegenerative diseases. WILSON et al:NEUROCHEMISTRY INTERNATIONAL, 50(7-8) 1-6-2007, p1067-1077, discloses that mice fed washed cycad flour containing three sterol glucosides known as neurotoxic in vitro, which provide a murine model of neurodegenerative disease, the cycad-induced model of ALS-parkinsonism dementia complex ; mice fed with the single purified beta-stosterol beta-D-glucoside (BSSG) neurotoxin of cycad exhibit significant motor neuron loss and other pathological markers but no significant behavioural deficits nor decrease GLT-1T density in the spinal cord. The inventors have discovered such animal models and methods for monitoring neurodegenerative diseases in animals using neurotoxic stigmasterol (SG) glucoside. A non-limiting list of illustrative neurodegenerative diseases that can be modeled using the compositions, methods and models described herein includes amyotrophic

lateral sclerosis, early onset Parkinson's disease, late onset Parkinson's disease, Alzheimer's disease, and the like.

Provided herein is an animal model of a neurodegenerative disease comprising a non-human animal that has been administered with a neurotoxic compound wherein the neurotoxic compound is synthetic and/or purified stigmasterol glucoside, and the non-human animal is a rodent.

Also provided herein is a method of monitoring a neurodegenerative disease in a non-human animal, the method comprising the steps of administering to the non-human animal a composition consisting essentially of a neurotoxic sterol glucoside; and monitoring the neurodegenerative disease in the non-human animal by using at least one behavioral abnormality test or at least one post-mortem test for neurodegeneration; wherein the neurotoxic sterol glucoside is synthetic and/or purified stigmasterol glucoside and the non-human animal is a rodent.

### BRIEF DESCRIPTION OF THE DRAWINGS

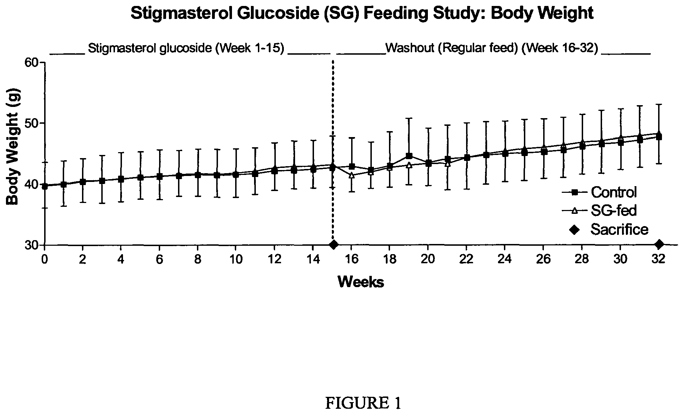

FIGURE 1. Body weights of SG-fed animals and their age-matched controls. A comparison of weight values between control and SG-fed animals revealed that body weights are not significantly different between groups and increase steadily over-time.

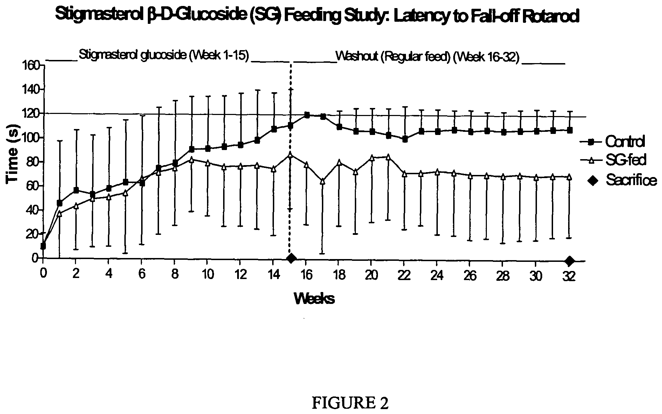

FIGURE 2. SG-feeding reduces rotarod performance. The rotarod test was used for the duration of the study to monitor motor coordination of the animals. SG-exposed animals demonstrated a trend of diminished performance on the rotarod but numbers did not reach statistical significance.

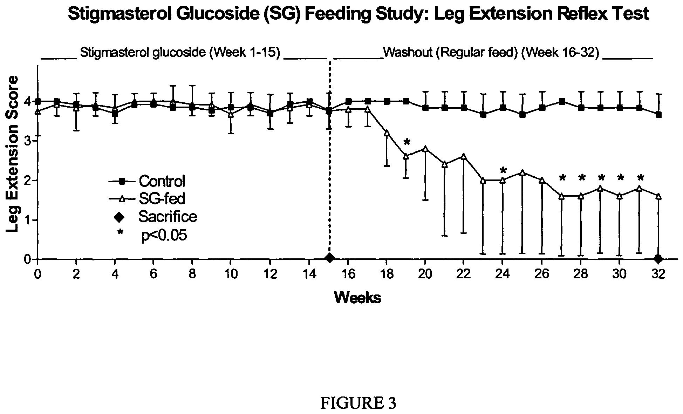

FIGURE 3. SG-feeding induces a decline in performance on the leg extension reflex test. The leg extension reflex test was used to monitor hind limb reflex in SG-exposed animals and their age-matched controls for the duration of the study. A steady decline in performance was evident amongst SG-fed animals soon after the first animal sacrifice (35 weeks). Time points at which the performance was found to be significant are marked with an asterisk (t-test: *p<0.05). In contrast, control animals showed no disturbance in hind limb reflex for the duration of the study.

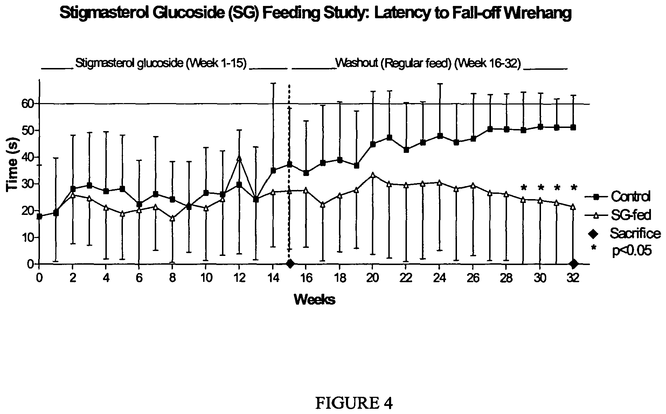

FIGURE 4. SG-feeding reduces performance in a forelimb strength analysis test. The wirehang test was used to monitor muscle strength in SG-fed animals and their age-matched controls for the duration of the study. A decline in performance was evident prior to the first animal sacrifice (35 weeks) and by fourteen weeks post-feeding, the difference in performance was statistically significant (t-test: *p<0.05) and remained so until the time the remaining mice were sacrificed.

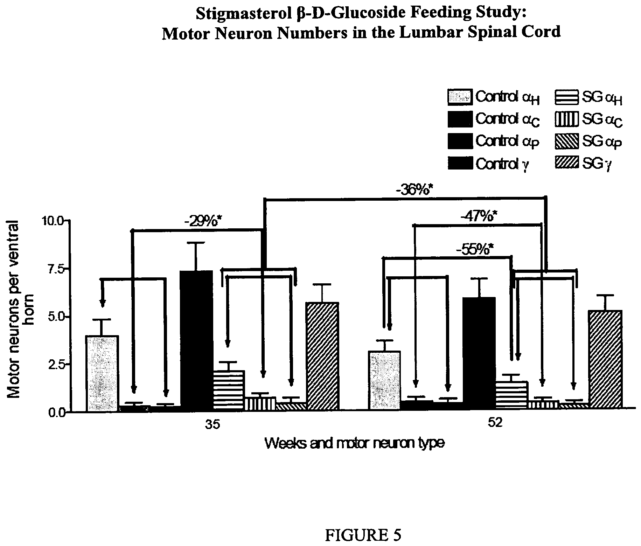

FIGURE 5. Motor neuron counts following SG-feeding. Motor neurons were differentiated with cresyl violet for Nissl bodies. Lumbar spinal cord α- and γ-motor neuron counts were conducted at 35 and 52 weeks (A). Motor neurons were sub-grouped into those with a normal, healthy appearance (subscript "H"), those that were chromatolytic (subscript "C") and those that were pyknotic (subscript "p"). At both 35 and 52 weeks, the control group was observed to have numerous healthy α- and γ- motor neurons in the ventral horn of the lumbar spinal cord. With the detailed motor neuron counts, statistical significance was detected between the number of "healthy" α- motor neurons between the control and SG-fed group at 52 weeks (t-test: -55%, p<0.05) only (A). When a statistical analysis was conducted between the total number of α-motor neurons between groups, a significant difference was detected at each of the time points (t-test at 35 weeks: -29%, *p<0.05; t-test at 52 weeks: - 47%, *p<0.05; red lines indicate subcategories that were grouped to obtain a α-motor neuron "total"). Further, the SG-fed group was found to undergo a significant decline in the total number of α-motor neurons between 35 and 52 weeks (t-test: -36%, *p<0.05; blue lines indicate the summation of subgroups in the graph).

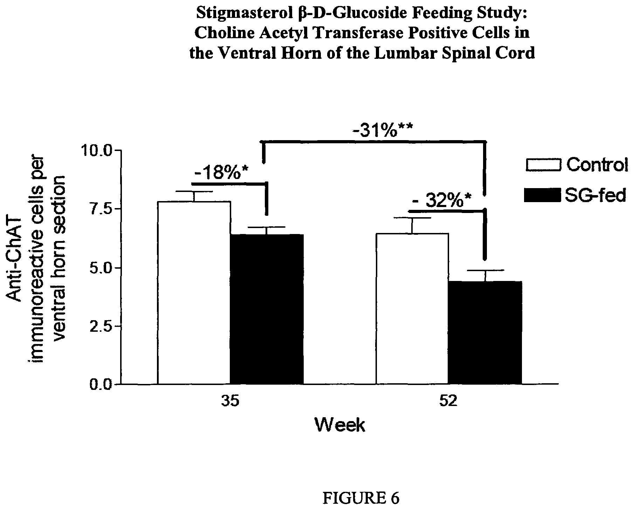

FIGURE 6. Cholinergic motor neurons in the spinal cord of SG-fed mice. Anti-choline acetyl transferase (ChAT) immunoreactive cells were counted as cholinergic cells. Quantification anti-ChAT labelling in the ventral horn of the lumbar spinal cord revealed a significant difference between the control and SG-fed groups at 35 and 52 weeks. SG-fed animals had 18% (t-test: *p<0.05) and 32% (t-test: *p<0.05) fewer cholinergic neurons at 35 and 52 weeks, respectively (A). Further, the SG-fed animals experienced a significant decrease in the number of cholinergic neurons between 35 and 52 weeks (t-test: - 31%, *p<0.05).

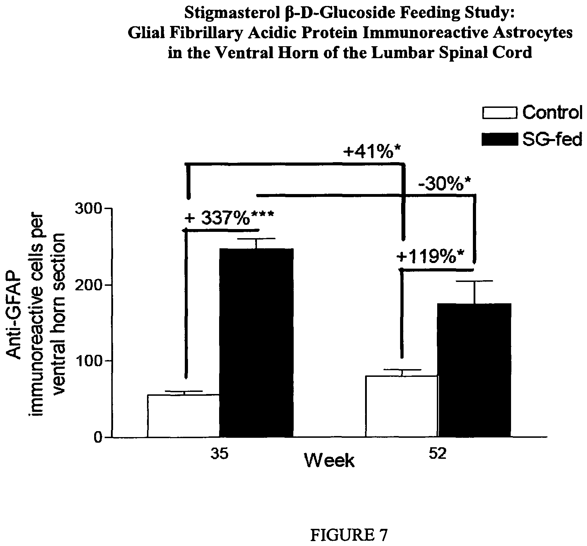

FIGURE 7. Activated astrocytes in the lumbar spinal cord of SG-fed mice. Anti-glial fibrillary acidic protein (GFAP) immunoreactive, stellate (both compact and elongated) cells were counted as astrocytes. Quantification of anti-GFAP labelling in the ventral horn of the lumbar spinal cord of control and SG-fed mice at 35 weeks and 52 weeks (A). SG-fed mice had a significantly greater number of astrocytic cells compared to the controls at both the pre-symptomatic (t-test: +337%, ***p<0.001) and symptomatic time-points (t-test: +119%, *p<0.05). Further, while the control animals experienced a significant increase in the number of astrocytes over time (t-test: +41%, *p<0.05), SG-fed animals were found to undergo a significant reduction in GFAP positive astrocytes (t-test: - 30%, *p<0.05) over the same 17 week period.

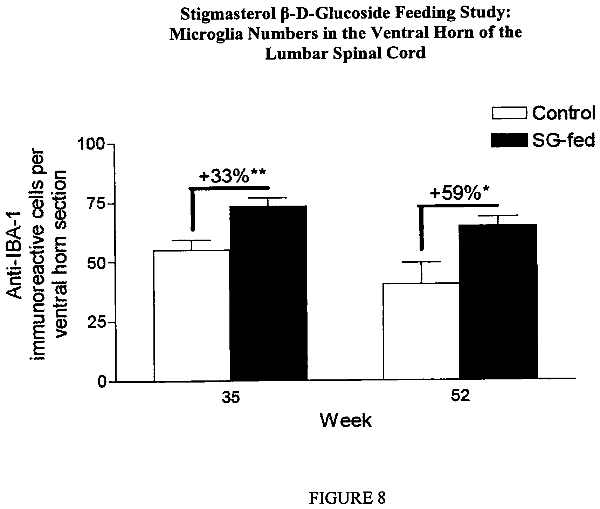

FIGURE 8. Microglial proliferation in the lumbar spinal cord of SG-fed mice. Cells immunoreactive to ionized calcium binding receptor-1 (IBA-1) were counted as microglia. (A) Quantification of microglia in the ventral horn of the lumbar spinal cord of control and SG-fed mice at 35 weeks and 52 weeks. SG-fed mice had significantly more microglia compared to the controls at both the pre-symptomatic (t-test: +33%, **p<0.01) and symptomatic (t-test: +59%, *p<0.05) time-points (Student's t-test, *p<0.05).

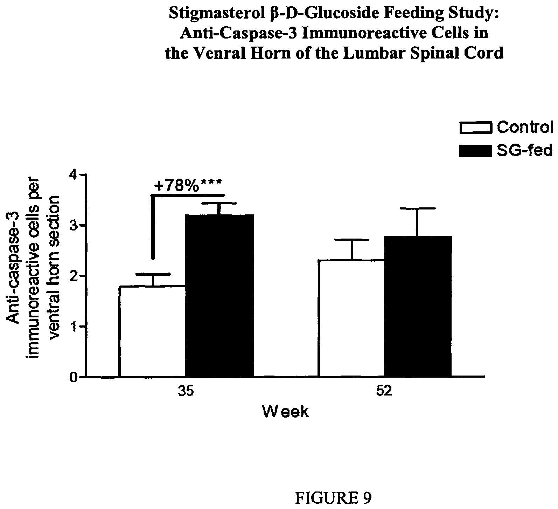

FIGURE 9. Caspase-3 labelling in the spinal cord of SG-fed mice. Quantification of anti-caspase-3 immunoreactive cells in the ventral horn of the lumbar spinal cord at 35 and 52 weeks revealed that SG-feeding induced cells to undergo apoptosis at pre-symptomatic and symptomatic time points. At 35 weeks, a significantly greater number of apoptotic neurons were observed in SG-fed animals (t-test, +78%, ***p<0.001) (A). A greater number of apoptotic cells were also observed in SG-fed animals at 52 weeks, but numbers did not reach significance (A).

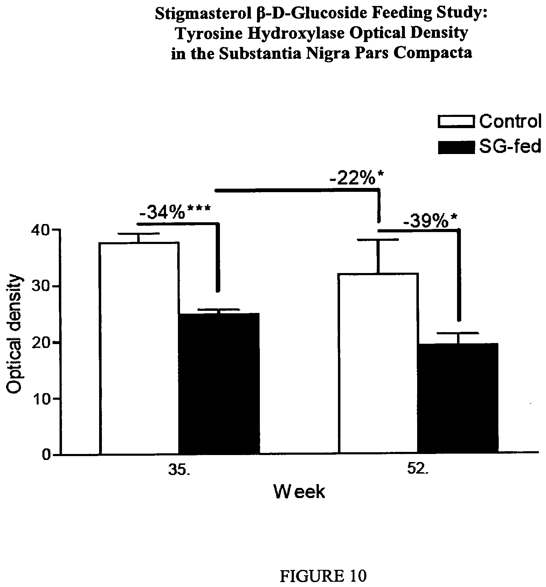

FIGURE 10. Dopaminergic cell labelling is lost in the striatum of SG-fed mice. SG-fed mice showed significantly decreased tyrosine hydroxylase labelling (optical density) compared to controls in the substantia nigra pars compacta (SNpc) at 35 weeks (t-test: -34%, ***p<0.001) and at 52 weeks (t-test: -39%, *p<0.05). TH optical density was found to decrease among SG-fed animals over time (t-test: -22%, *p<0.05) in the SNpc (A). A decrease in optical density was not found among the striata of the SG-fed animals' at either time-point (t-test: not significant p=0.0846) (F). The toxicity to motor and niagral striata caused in SG-fed mice indicates that SG can be used in models of motor neuron disease and in Parkinson's disease models, among other neurodegenerative disease states.

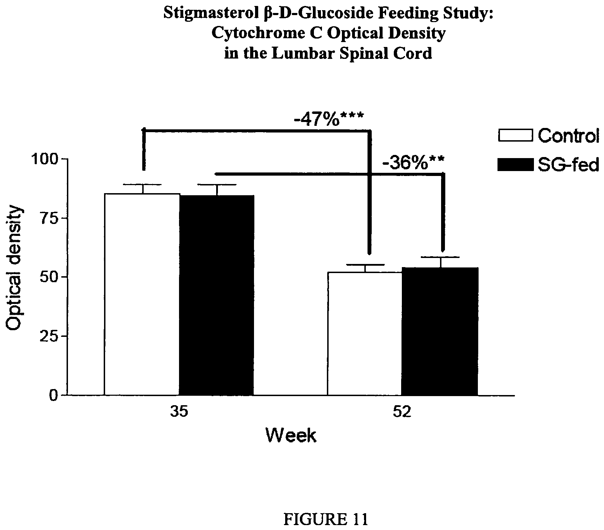

FIGURE 11. Cytochrome c oxidase activity is unaffected in SG-fed animals. Cytochrome c oxidase activity was analyzed in SG-fed animals and age-matched controls. No significant differences were observed between groups at 35 or 52 weeks (A). A decline in cytochrome c oxidase activity was observed to occur over time in each of the groups (A).

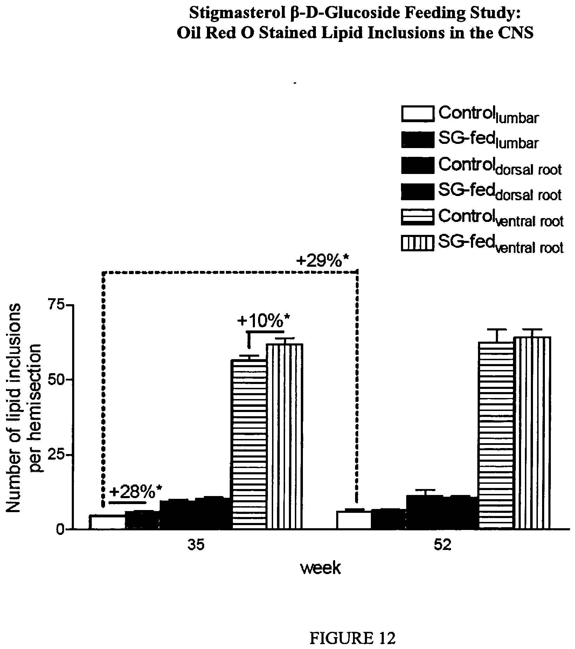

FIGURE 12. Oil red O staining of lipids in the lumbar spinal cord and dorsal and ventral spinal roots. Lipid deposits in various CNS regions were revealed by oil red O (red coloured stain). Nuclei were stained with haematoxylin (blue/purple coloured stain). Lipid deposits were observed in the motor neurons of control and SG-fed mice, but the number and size of the deposits were greater in lumbar spinal cord and ventral roots of SG-fed animals. Panel A shows a quantification of lipid deposits in control and SG-fed animals at 35 and 52 weeks. An inter-group assessment of the ventral horn of the lumbar cord revealed that at 35 weeks, SG-fed animals had significantly more lipid deposits in the ventral horn of the lumbar spinal cord (Student's t-test, +28%, *p<0.05). SG-fed animals also had significantly more lipids in the ventral spinal roots compared to control animals at 35 weeks (Student's t-test: +10%, *p<0.05). A similar comparison of the dorsal roots did not show a statistically significant difference (Student's t-test: not significant, p=0.3778). At 52 weeks, statistically significant inter-group differences were not found in the three CNS regions that were assessed (lumbar spinal cord: Student's t-test, p=0.6406; dorsal root: Student's t-test, p=0.6958; ventral root: Student's t-test, p=0.7422). Time-dependent effect assessment of lipid accumulation in SG-fed animals and age-matched controls was also performed. Control animals showed a statistically significant increase in the total number of lipid deposits over time (Student's t-test: +29%, p<0.05). SG-fed animals did not experience an accumulation of deposits over time (Student's t-test, p=0.4864). Both SG-fed animals and controls did not show time-dependent effects in the dorsal (Student's t-tests- control: p=0.4864; SG-fed: p=0.5478) or ventral roots (Control: p=0.1301; SG-fed: p=0.5478).

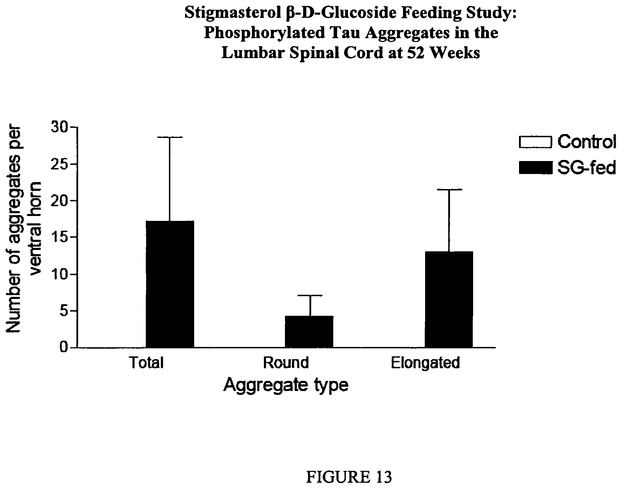

FIGURE 13. Select SG-fed mice accumulated phosphorylated tau in the ventral horn of the lumbar spinal cord and a subset of these phosphorylated tau-containing cells underwent apoptosis. Dietary SG-feeding induced accumulation of phosphorylated tau. The majority of the phosphorylated-tau containing cells were actively undergoing apoptosis in animals at 52 weeks. (A) The numbers of elongated and round aggregates were quantified separately (A).

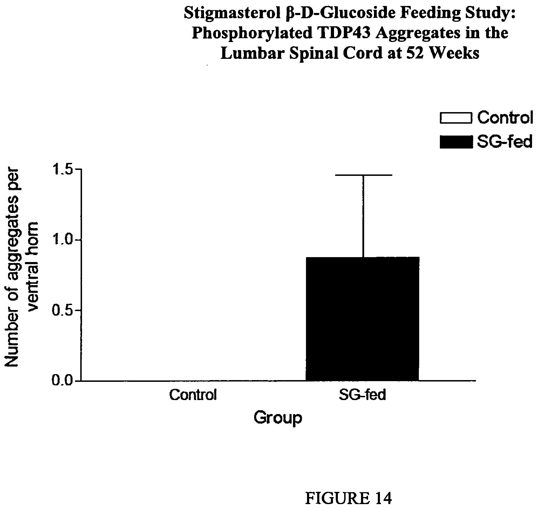

FIGURE 14. TDP-43 pathology occurs in the lumbar spinal cord of a subset of mice fed SG. Two of eight mice fed SG, survived to 52 weeks exhibited TAR DNA binding protein (TDP) 43 pathology. In healthy cells, TDP-43 is found in the nucleus, but redistributes and accumulates in the cytosol in neurological conditions, such as in sporadic ALS. No abnormal translocation of TDP-43 was observed in control animals, SG-fed animals at 35 weeks, and in the majority of SG-fed animals at 52 weeks. The frontal cortex, hippocampus, and motor cortex were also negative in control animals at both time-points. Panel A depicts quantification of pathologic phosphorylated TDP-43-containing aggregates in the lumbar spinal cord. These aggregates increased in the SG-fed animals.

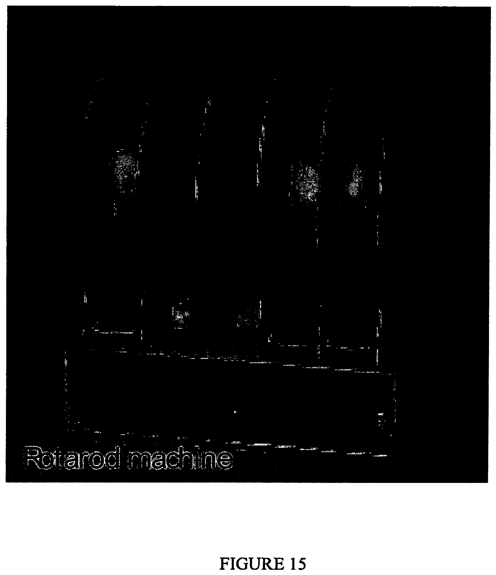

FIGURE 15. A rotorod apparatus is used to measure the time mice can remain walking on a horizontal, rotating axle (3.6 cm diameter; 16 rpm) elevated above an area of padding without falling, clenching on, or jumping off the axle.

### DETAILED DESCRIPTION

The invention in its broadest sense is as defined in the independent claims. The invention provides an animal model of a neurodegenerative disease comprising a non-human animal that has been administered with a neurotoxic compound wherein the neurotoxic compound is synthetic and/or purified stigmasterol glucoside, and the non-human animal is a rodent. The invention further provides a method of monitoring a neurodegenerative disease in a non-human animal, the method comprising the steps of administering to the non-human animal a composition consisting essentially of a neurotoxic sterol glucoside; and monitoring the neurodegenerative disease in the non-human animal by using at least one behavioral abnormality test or at least one post-mortem test for neurodegeneration;wherein the neurotoxic sterol glucoside is synthetic and/or purified stigmasterol glucoside and the non-human animal is a rodent. In one illustrative example described herein is an animal model of a neurodegenerative disease comprising a non-human animal in contact with a neurotoxic compound of formula 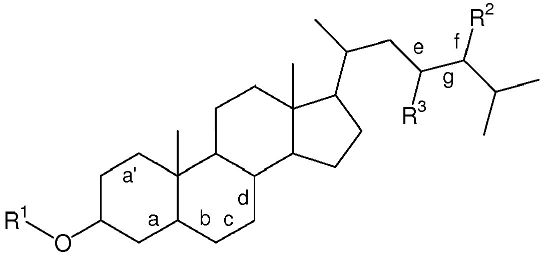
wherein R¹ is optionally substituted glycosyl;
R² is hydrogen, alkyl, alkenyl, alkynyl, heteroalkyl, haloalkyl, or OR⁴;
R³ is hydrogen, alkyl, alkenyl, heteroalkyl, haloalkyl, or OR⁴;
R⁴ is in each instance independently selected from the group consisting of hydrogen, alkyl, heteroalkyl, haloalkyl, alkenyl, alkynyl, and acyl;
the bonds labeled a, a', b, c, d, e, f, and g are each independently selected from the group consisting of a single bond and a double bond, with the proviso that for the bonds labeled b, c, and d, adjacent bonds are not both double bonds; and wherein the neurotoxic compound is not a compound selected from the group consisting of β-sitosterol-β-D-glucoside and cholesterol glucoside.

In another example described herein is the model of any one of the preceding examples wherein R¹ is selected from the group consisting of hexosyl and pentosyl, each of which is optionally substituted; R² is methyl or ethyl; and R³ is hydrogen.

In another example described herein is the model of any one of the preceding examples wherein R¹ is selected from the group consisting of hexosyl and pentosyl, each of which is optionally substituted; R² is methyl or ethyl; R³ is hydrogen; and the bond labeled b is a double bond.

In another example described herein is the model of any one of the preceding examples wherein the bond labeled g is a double bond.

In another example described herein is the model of any one of the preceding examples wherein R¹ has formula 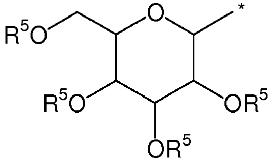 wherein R⁵ is in each instance independently selected from the group consisting of hydrogen, methyl and C(O)R⁶, where R⁶ is alkyl, haloalkyl, or heteroalkyl.

In another example described herein is the model of any one of the preceding examples wherein the R¹ is β-D-glucosyl. In another example described herein is the model of any one of the preceding exampleswherein the neurotoxic compound is selected from the group consisting of campesterol glucoside, dihydrobrassicasterol glucoside, ergosterol glucoside, and avenasterol glucoside. In another example described herein is the model of any one of the preceding examples wherein the neurotoxic compound is a mixture of two or more of campesterol glucoside, dihydrobrassicasterol glucoside, stigmasterol glucoside, ergosterol glucoside, avenasterol glucoside, or sitosterol glucoside.

In another example the neurotoxic compound is campesterol glucoside. In another example the neurotoxic compound is dihydrobrassicasterol glucoside. The non-human animal used in the animal model of neurodegenerative disease can be any suitable laboratory animal. The non-human animal of the invention is a rodent, such as a mouse, a rat, a hamster, a guinea pig, a gerbil, and the like. In other aspects, the non-human animal can be a primate, for example, a monkey, a chimpanzee, a macaque, a squirrel monkey, an ape, or any other primate useful in an animal model of neurodegenerative disease. In other illustrative examples, the non-human animal can be a rabbit, a dog, a cat, or any other suitable laboratory animal. The neurodegenerative disease can be selected from a variety of disease states. The neurodegenerative disease can be selected from the group consisting of progressive supranuclear palsy, Alzheimer's disease, frontotemporal dementia (e.g., Pick's disease, frontotemporal lobar degeneration, progressive aphasia, and semantic dementia), motor neuron disease (e.g., amyotrophic lateral sclerosis, primary lateral sclerosis, progressive bulbar palsy, pseudobulbar palsy, progressive muscular atrophy, and spinal muscular atrophy), neuropathy, peripheral neuropathy, early onset Parkinson's disease, and late onset Parkinson's disease. In an embodiment of the model of the invention the neurodegenerative disease is selected from the group consisting of amyotrophic lateral sclerosis, early onset Parkinson's disease, late onset Parkinson's disease, and Alzheimer's disease. In yet another aspect the neurodegenerative disease is Alzheimer's disease or Parkinson's disease. Alternatively, in any one of the preceding embodiments the neurodegenerative disease can be amyotrophic lateral sclerosis. In another embodiment, neurodegeneration in general can be studied.

In yet another example described herein, a method of monitoring a neurodegenerative disease in a non-human animal is provided. The method comprises the steps of administering to the non-human animal a composition comprising a neurotoxic compound of formula 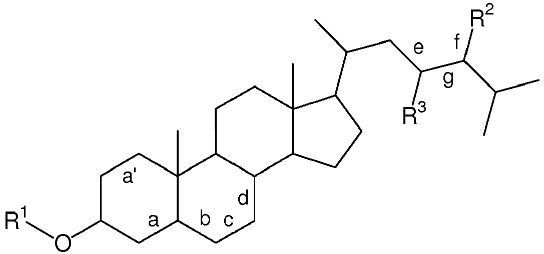
wherein R¹ is optionally substituted glycosyl;
R² is hydrogen, alkyl, alkenyl, alkynyl, heteroalkyl, haloalkyl, or OR⁴;
R³ is hydrogen, alkyl, alkenyl, heteroalkyl, haloalkyl, or OR⁴;
R⁴ is in each instance independently selected from the group consisting of hydrogen, alkyl, heteroalkyl, haloalkyl, alkenyl, alkynyl, and acyl;
the bonds labeled a, a', b, c, d, e, f, and g are each independently selected from the group consisting of a single bond and a double bond, with the proviso that for the bonds labeled b, c, and d, adjacent bonds are not both double bonds; and wherein the compound is not a compound selected from the group consisting of β-sitosterol-β-D-glucoside and cholesterol glucoside; and monitoring the neurodegenerative disease in the non-human animal.

In another example described herein is the method of any one of the preceding examples wherein R¹ is selected from the group consisting of hexosyl and pentosyl, each of which is optionally substituted.

In still another example described herein is the method of any one of the preceding examples wherein R¹ is selected from the group consisting of hexosyl and pentosyl, each of which is optionally substituted; R² is methyl or ethyl; and R³ is hydrogen.

In yet another example described herein is the method of any one of the preceding examples wherein R¹ is selected from the group consisting of hexosyl and pentosyl, each of which is optionally substituted; R² is methyl or ethyl; R³ is hydrogen; and the bond labeled b is a double bond.

In another example described herein is the method of any one of the preceding examples wherein the bond labeled g is a double bond.

In another example described herein is the method of any one of the preceding examples wherein R¹ has formula 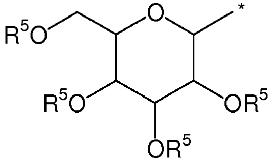 wherein R⁵ is in each instance independently selected from the group consisting of hydrogen, methyl and C(O)R⁶, where R⁶ is alkyl, haloalkyl, or heteroalkyl.

In still another example described herein is the method of any one of the preceding examples wherein the R¹ is β-D-glucosyl. In an alternate example described herein is the method of any one of the preceding examples wherein the neurotoxic compound is selected from the group consisting of campesterol glucoside, dihydrobrassicasterol glucoside, ergosterol glucoside, and avenasterol glucoside. Further illustratively, in the method of any one of the preceding examples the neurotoxic compound can be a mixture of two or more of campesterol glucoside, dihydrobrassicasterol glucoside, stigmasterol glucoside, ergosterol glucoside, avenasterol glucoside, or sitosterol glucoside. In the method of the invention the neurotoxic compound is stigmasterol glucoside. In another example described herein is the method of any one of the preceding examples wherein the neurotoxic compound is campesterol glucoside. In another method example the neurotoxic compound can be dihydrobrassicasterol glucoside. The non-human animal used in the method of monitoring a neurodegenerative disease can be any suitable laboratory animal. The non-human animal can be a rodent, such as a mouse (e.g. CD-1 mice, Charles River), a rat (e.g., Sprague-Dawley rats, Charles River), a hamster, a guinea pig, a gerbil, and the like. In other examples, the non-human animal can be a primate, such as a monkey, a chimpanzee, a macaque, a squirrel monkey, an ape, or any other primate useful in the method of monitoring a neurodegenerative disease. In other illustrative examples, the non-human animal can be a rabbit, a dog, a cat, or any other suitable laboratory animal. The neurodegenerative disease can be selected from a variety of disease states. The neurodegenerative disease in the methods described herein can be selected from the group consisting of progressive supranuclear palsy, Alzheimer's disease, frontotemporal dementia (e.g., Pick's disease, frontotemporal lobar degeneration, progressive aphasia, and semantic dementia), motor neuron disease (e.g., amyotrophic lateral sclerosis, primary lateral sclerosis, progressive bulbar palsy, pseudobulbar palsy, progressive muscular atrophy, and spinal muscular atrophy), neuropathy, peripheral neuropathy, early onset Parkinson's disease, and late onset Parkinson's disease. In an embodiment of the method described herein, the neurodegenerative disease can be selected from the group consisting of amyotrophic lateral sclerosis, early onset Parkinson's disease, late onset Parkinson's disease, and Alzheimer's disease. In yet another aspect the neurodegenerative disease is Alzheimer's disease or Parkinson's disease. Alternatively, in any one of the preceding method aspects the neurodegenerative disease can be amyotrophic lateral sclerosis. In another aspect neurodegeneration in general can be studied. The non-human animal can be put in contact with the neurotoxic compound or the neurotoxic compound can be administered to the non-human animal by methods well-known to those skilled in the art. For example, the composition comprising the neurotoxic compound can be administered to the non-human animal or the non-human animal can be put in contact with the neurotoxic compound by modifying the non-human animal's daily diet by adding the neurotoxic compound or the composition comprising the neurotoxic compound to the diet and feeding the diet to the non-human animal. In addition to the neurotoxic compound or the composition comprising the neurotoxic compound, other additives can be used to further modify any standard diet for the non-human animal. An illustrative non-limiting list of additives is vitamins, minerals (e.g., calcium and phosphorus), amino acids, emulsifying agents, flavorings, flavor-masking agents, fragrances, antibiotics, anti-parasiticides, anti-nausea agents, protein, fat, fiber, and combinations thereof.

Standard diets for laboratory animals are well-known in the art and, illustratively, any standard pelleted, grain-based diet can be used (e.g., Rodent Diet 20, PicoLab; Teklad Rodent Diet #8064, Harlan Teklad, Madison, Wis.; Mouse Diet™, Purina, St.Louis Mo; Prolab Rat, Mouse, Hamster 3000, St.Louis Mo.). The animals are typically allowed free access to water.

In other examples the non-human animal can be put in contact with the neurotoxic compound or the neurotoxic compound can be administered by gavage feeding or parenteral injection in any acceptable carriers such as liquid alcohols, glycols (*e.g.,* polyethylene glycols), glucose solutions (*e.g*., 5%), esters, amides, sterile water, or buffered saline (including buffers like phosphate or acetate; *e.g.*, isotonic saline). The neurotoxic compound can also be administered in the form of a pill, a paste, or a tablet, for example. Additional exemplary components that can be included in the composition along with the neurotoxic compound are vegetable oils, gelatin, lactose, amylose, magnesium stearate, solubilizing agents, local anaesthetics (*e.g*., lidocaine), excipients, preservatives, stabilizers, wetting agents, emulsifiers, salts, and lubricants, depending on the dosage form of the neurotoxic compound.

In another example the composition comprising the neurotoxic compound can be injected parenterally and such injections can illustratively be intraperitoneal injections, subcutaneous injections, intramuscular injections, or intravenous injections. In another example the composition comprising the neurotoxic compound can be delivered using a slow pump. Examples of parenteral dosage forms include aqueous solutions of the active agent in well-known pharmaceutically acceptable liquid carriers such as liquid alcohols, glycols *(e.g.,* polyethylene glycols), glucose solutions (*e.g.,* 5%), esters, amides, sterile water, or buffered saline (including buffers like phosphate or acetate; *e.g*., isotonic saline). In another illustrative aspect, the parenteral dosage form can be in the form of a reconstitutable lyophilizate comprising the dose of the neurotoxic compound. In various aspects, solubilizing agents, local anaesthetics *(e.g.,* lidocaine), excipients, preservatives, stabilizers, wetting agents, emulsifiers, salts, and lubricants can be used.

In various aspects the dose of the neurotoxic compound for any of the preceding embodiments can be in the range of about 0.1 mg/kg of animal body weight per day to about 500 mg/kg of animal body weight per day. In another embodiment, the dose of the neurotoxic compound can be in the range of about 1.0 mg/kg of animal body weight per day to about 400 mg/kg of animal body weight per day. In other embodiments, the dose of the neurotoxic compound can be in the range of about 10 mg/kg of animal body weight per day to about 300 mg/kg of animal body weight per day, about 10 mg/kg of animal body weight per day to about 200 mg/kg of animal body weight per day, about 10 mg/kg of animal body weight per day to about 100 mg/kg of animal body weight per day, or about 10 mg/kg of animal body weight per day to about 50 mg/kg of animal body weight per day. The composition comprising the neurotoxic compound can be administered as single doses, or the doses can be divided and administered as a multiple-dose daily regimen. Further, a staggered regimen, for example, one to six days per week, once per week, or one to three times per month can be used as an alternative to daily administration. Also, daily administration is suitable.

In an embodiment, the method of wherein the neurodegenerative disease is monitored or studied uses any one or more of the tests selected from the group consisting of a leg extension test, a gait length test, a rotarod test, a wire hang test, a water maze test, a radial arm maze test, a Digigate system test, an Ethovision test, an anxiety test, a depression test, and an olfactory system test. The procedures for conducting these tests for monitoring and studying neurodegeneration and neurodegenerative diseases are described herein in the EXAMPLES and are well-known to those skilled in the art. Methods for determining and measuring *in vivo* and *in vitro* neurotoxic effects on cells and tissues of the nervous system of non-human animals and for studying the behavior of non-human animals exposed to the neurotoxic compounds and compositions described herein are detailed in WO200237122. The disclosure also described is a neurotoxic composition for use in a model of a neurodegenerative disease comprising a compound of formula 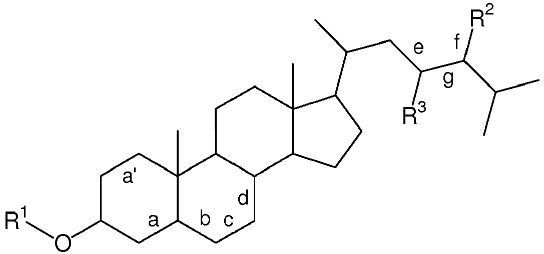 wherein
R¹ is optionally substituted glycosyl;
R² is hydrogen, alkyl, alkenyl, alkynyl, heteroalkyl, haloalkyl, or OR⁴;
R³ is hydrogen, alkyl, alkenyl, heteroalkyl, haloalkyl, or OR⁴;
R⁴ is in each instance independently selected from the group consisting of hydrogen, alkyl, heteroalkyl, haloalkyl, alkenyl, alkynyl, and acyl;
the bonds labeled a, a', b, c, d, e, f, and g are each independently selected from the group consisting of a single bond and a double bond, with the proviso that for the bonds labeled b, c, and d, adjacent bonds are not both double bonds; and wherein the compound is not a compound selected from the group consisting of β-sitosterol-β-D-glucoside and cholesterol glucoside; and a pharmaceutically acceptable carrier therefore.

In another example described herein is the neurotoxic composition wherein R¹ is selected from the group consisting or hexosyl and pentosyl, each of which is optionally substituted.

In another example described herein is the neurotoxic composition wherein R¹ is selected from the group consisting of hexosyl and pentosyl, each of which is optionally substituted; R² is methyl or ethyl; and R³ is hydrogen.

In another example described herein is the neurotoxic composition wherein R¹ is selected from the group consisting or hexosyl and pentosyl, each of which is optionally substituted; R² is methyl or ethyl; R³ is hydrogen; and the bond labeled b is a double bond.

In another example described herein is the neurotoxic composition wherein the bond labeled g is a double bond.

In another example described herein is the neurotoxic composition of any one of the preceding embodiments wherein R¹ has formula 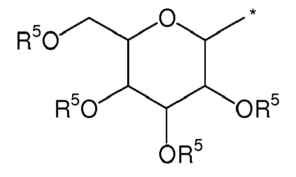 wherein R⁵ is in each instance independently selected from the group consisting of hydrogen, methyl and C(O)R⁶, where R⁶ is alkyl, haloalkyl, or heteroalkyl.

In another example described herein is the neurotoxic composition wherein the R¹ is β-D-glucosyl. In another example the neurotoxic composition is selected from the group consisting of campesterol glucoside, dihydrobrassicasterol glucoside, ergosterol glucoside, and avenasterol glucoside. In another example the neurotoxic composition is a mixture of two or more of campesterol glucoside, dihydrobrassicasterol glucoside, stigmasterol glucoside, ergosterol glucoside, avenasterol glucoside, or sitosterol glucoside.

In another example of neurotoxic composition the neurotoxic compound is campesterol glucoside. In another example of neurotoxic compo the neurotoxic compound is dihydrobrassicasterol glucoside.

The neurodegenerative disease can be selected from a variety of disease states. The neurodegenerative disease can be selected from the group consisting of progressive supranuclear palsy, Alzheimer's disease, frontotemporal dementia (e.g., Pick's disease, frontotemporal lobar degeneration, progressive aphasia, and semantic dementia), motor neuron disease (e.g., amyotrophic lateral sclerosis, primary lateral sclerosis, progressive bulbar palsy, pseudobulbar palsy, progressive muscular atrophy, and spinal muscular atrophy), neuropathy, peripheral neuropathy, early onset Parkinson's disease, and late onset Parkinson's disease. In another embodiment of the compositions described herein, the neurodegenerative disease can be selected from the group consisting of amyotrophic lateral sclerosis, early onset Parkinson's disease, late onset Parkinson's disease, and Alzheimer's disease. The neurotoxic composition can be in a form for gavage feeding or parenteral injection in any acceptable carriers such as liquid alcohols, glycols (*e.g.,* polyethylene glycols), glucose solutions (*e.g.,* 5%), esters, amides, sterile water, or buffered saline (including buffers like phosphate or acetate; e.g., isotonic saline). The neurotoxic composition can also be in the form of a pill, a paste, or a tablet, for example. Additional exemplary components that can be included in the composition along with the neurotoxic compound are vegetable oils, gelatin, lactose, amylose, magnesium stearate, solubilizing agents, local anaesthetics (*e.g*., lidocaine), excipients, preservatives, stabilizers, wetting agents, emulsifiers, salts, and lubricants, depending on the dosage form of the neurotoxic composition. In accordance with this disclosure the phrase "pharmaceutically acceptable carrier" means any of these carriers or components or any other pharmaceutically acceptable carriers known in the art. The composition comprising the neurotoxic compound can be administered as single doses, or the doses can be divided and administered as a multiple-dose daily regimen. Further, a staggered regimen, for example, one to six days per week, once per week, or one to three times per month can be used as an alternative to daily administration. Also, daily administration is suitable.

In one illustrative example the neurotoxic compound is synthetic. In one aspect of the invention, the neurotoxic compounds described herein can be prepared by methods known in the art from commercially available sterols. An illustrative example is disclosed in US 2006/0252705A1. In another aspect the neurotoxic compound is purified. Purification of the neurotoxic compound can be carried out by any conventional techniques known to those skilled in the art, such as, gel filtration, ion exchange chromatography, column chromatography, affinity chromatography, solvent-solvent extraction, molecular distillation, crystallization, ultrafiltration, and HPLC. Illustratively, the purification of β-sitosterol β-D-glucoside, campesterol β-D-glucoside, dihydrobrassicasterol β-D-glucoside, stigmasterol β-D-glucoside, and b-sitosterol β-D-glucoside is described in Khabazian, I., et al, Journal of Neurochemistry, 2002, 82, 516-528; the disclosure of which is incorporated by reference herein in its entirety. The neurotoxic compound can be greater than 90%, 95%, 96%, 97%, 98%, 98.5%, 99%, 99.5%, or 99.7% pure, for example.

Certain of the compounds described herein contain one or more chiral centers, or may otherwise be capable of existing as multiple stereoisomers. Are included pure stereoisomers as well as mixtures of stereoisomers, such as enantiomers, diastereomers, and enantiomerically or diastereomerically enriched mixtures. Certain of the compounds described herein may be capable of existing as geometric isomers. Are included pure geometric isomers or mixtures of geometric isomers.

One skilled in the art will also readily recognize that where members are grouped together in a common manner, such as in a Markush group, are encompassed not only the entire group listed as a whole, but each member of the group individually and all possible subgroups of the main group, but also the main group absent one or more of the group members and the explicit exclusion of one or more of any of the group members.

### EXAMPLE 1

### ANIMALS

### Mice:

Five-month old male CD-1 mice (30.1-35.7 g, 7.5 cm - 8.8 cm) were purchased from Charles River (Wilmington, MA). Adult mice were chosen to mimic the mean age of onset of ALS-PDC (Kurland, 1988; Kurland et al., 1994). Thirty mice for the SG study were randomly divided into 2 groups (14 control and 16 experimental animals). All animals were housed singly in a virus-free barrier facility in a room maintained at 22°C under a 12 hour light/dark cycle. The mice were provided *ad libitum* access to food and water.

### EXAMPLE 2

### FEEDING PROTOCOLS

### Feeding:

The synthetic SG was mixed with finely ground Purina Chow mouse pellets (Mouse Diet™, Purina) to create the experimental pellets with a SG content of 1000 µg per pellet. Dry materials were blended with a sterile silicon spatula. For every 5 g of standard feed, 2.5 mL of doubly-distilled water (dd)H2O was added and the dough was kneaded and round up into pellets. A single sterol glucoside containing pellet was placed in the feeding tray each morning before the addition of regular mouse chow (*ad libitum).* All experimental pellets were entirely consumed by the mice each day. Control mice were fed standard mouse chow pellets of identical weight with no additives. SG feeding was conducted daily for 15 weeks and mice were behaviorally monitored until sacrifice at 35 weeks (time point 1) or 52 weeks (time point 2). The animals in both groups showed normal weight gain. See FIGURE 1.

### EXAMPLE 3

### BEHAVIORAL TESTS

### Behavioral Monitoring:

General conditions: The mice were tested singly in each of the behavioral experiments between 10 a.m. and 5 p.m. under standard light conditions (Crawley, 2000) in the virus-free facility. The testing sequence of mice was randomized across groups at every session. The experimental group to which each animal belonged was unknown to the observer at the time of behavioral recording.

Rotorod: The rotarod was used to evaluate motor function, coordination and balance (Jones and Roberts, 1968; Sango et al., 1995; Gerlai et al., 1996a; Sango et al., 1996; Chapillon et al., 1998; Carter et al., 1999; Crawley, 1999; Gerlai and Roder, 1996). The time that a mouse could remain walking on a horizontal (Norflus et al., 1998), rotating axle (3.6 cm diameter; 16 rpm) elevated above an area of padding without falling, clenching on, or jumping off the axle was measured (Crawley, 1999), see FIGURE 15. Each mouse was placed in the center of the rod (Chen et al., 1995) and tested for a maximum of 160 seconds (Barneoud et al., 1997; Crawley, 1999) twice per week. The SG fed mice showed a decrease in the time without fall, see FIGURE 2.

Wire hang: Neuromuscular strength was tested by the wire hang (or hanging wire) test (Wilson et al., 2003d; Wilson et al., 2004c; Wilson et al., 2005; Tabata et al., 2008). Both balance and grip strength are required for a mouse to keep its body suspended (Crawley, 2000). Mice were placed on a wire cage lid elevated 50 cm above a soft landing surface. The latency to fall off the wire (maximum 60 seconds) (Sango et al., 1996) was measured from the moment the wire apparatus was inverted (180°). Wire hang testing was conducted biweekly. The control group showed an increase in the time between inversion of the apparatus and falling, while the SG fed mice did not. See FIGURE 4.

Leg extension reflex test: The leg extension reflex test was used as a measure of motor neuron dysfunction (Barneoud and Curet, 1999). An altered form of this was used to discriminate more subtle behavioral changes. A 0-4 scale developed with consideration of possible animal responses was utilized (Wilson et al., 2002a): A score of 4 was assigned to an animal that exhibited complete extension of both legs. Such a response was considered a normal response. A score of 3 was assigned when an animal extended both legs with some tremors and/or punching of one leg. A score of 2 indicated that the animal extended one leg and retracted the other, or that the animal exhibited tremors in both legs. A score of 1 indicated that the animal retracted one leg and exhibited tremors in the other leg. A score of 0 was assigned when both legs were retracted. The leg extension reflex was measured three times per week in each animal. This scaled test was designed to show the progressive loss of function as the normal reflex has been found to deteriorate progressively to tremor and then to total retraction in previous cycad and sterol glucoside studies (Wilson et al., 2002b; Tabata et al., 2008). The leg extension score decreased for the SG-fed mice during the study, see FIGURE 3.

Open field monitoring: Spontaneous activity in an open field is considered one of the most standardized general measures of motor function (Crawley, 2000). The open field test can also reveal stress response in animals (i.e. defensive burying or thigmotaxis) (Karl et al., 2003). Open field testing of each animal was conducted every two weeks. A single mouse was placed in one of four round open field arena/tubs (approximately 1 m diameter) to permit the testing of four animals per 5 minute trial. Five minutes has been shown to be sufficient for evaluation of gross abnormalities in locomotion and highly significant hyperactivity or behavioral sedation (Crawley, 2000). Large arenas were employed since they have better defined "regions" (i.e. center and perimeter) and permit mice to cover longer distances and demonstrate a wider variation in movement. This in turn, increases the likelihood of detection of fear-based behavior (Crawley, 2000) and minimizes habituation of the animal to the open field (Crawley, 2000). Testing arenas were cleaned with 70% ethanol before the testing of each animal and with quatricide at the end of each testing session. Movements were recorded using a video camera mounted on the ceiling. Video clips were reviewed and were manually scored or automatically analysed on an automated video tracking software (Ethovision® 3.1, Noldus Information Technologies Inc., Leesburg, VA, USA). Scoring was done on videotaped sessions, rather than in-person at the time of testing to avoid the introduction of confounding variables by the presence of an observer in close proximity to the mice (Crawley, 2000).

Gait analysis: In order to examine spatial and temporal indices of front and hind limb stepping patterns during forward locomotion, mice were required to run on a colorless (PVC and polycarbonate), straight, level, walled motorized belt (Digigait™, treadmill motor: DC, HP; torque 45 lb-in, Mouse Specifics, Inc., Boston, MA, USA) set at 30 rpm with a no-flicker light source (90-250 VAC; 25 kHZ, Mouse Specifics, Inc., Boston, MA, USA) and camera mounted ventrally (relative to the animal) to facilitate continuous ventral plane videography (up to 150 frames per second) of activity. Gait indices were analyzed with the complementary digital imaging software (Digigait™, Mouse Specifics, Inc., Boston, MA, USA) as per manufacturer's instructions. Briefly, the software converts "paw prints" into a digital signal and generates a temporal record of paw placement and breaks down the digital file into three main parameters: stride duration, swing duration, and stance duration. The stance duration is the period during which the paw of a limb is in contact with the belt surface. The software further subdivides the stance duration into two components: braking duration (the increase in paw contact area over a discrete time interval) and propulsion duration (the decrease in paw contact area over a discrete time interval). The swing duration is the period of time that a paw is not in contact with the belt surface. In addition to these factors, over a dozen additional characteristics, including stance factor, step-sequence pattern, propulsion, and cadence are generated to provide a spreadsheet format report of twenty-five gait indices.

### EXAMPLE 4

### ORGAN HARVESTING AND CRYOSECTIONING

Mice were sacrificed for histological analysis at 35 weeks (35.9-48.6 g) and 52 weeks (41.2-51.4 g) of age. Age-matched control mice from each time point were included for comparison. The mice were deeply anaesthetized in a chamber with 4% isoflurane and perfused intracardially with filtered PBS (pH 7.4) until the effluent was cleared of blood (2 minutes), followed by perfusion with cold (4°C) 4% paraformaldehyde (PFA) in 0.1 M phosphate buffer (pH 7.4) for 5 minutes (30 mL). The PBS and the PFA were delivered through separate sterile platinum-cured silicon tubing (Cole-Parmer Canada Inc., Montreal, Canada) and peristaltic pumps (MasterFlex peristaltic pump, Cole-Parmer Canada Inc., Montreal, Canada). The brains and spinal cords were dissected out and fixed overnight at 4°C and equilibrated in increasing concentrations of sucrose in phosphate-buffered saline, pH 7.4 (10, 20, and 25%) over a 3-day period. Complete equilibration of the tissues was taken as the moment the tissues sank to the bottom of the scintillation tubes filled with 25% sucrose. After equilibration, the spinal cord and brain specimens were set in molds (Tissue-tek Cryomold, Sakura Finetek, USA, Torrance, CA) with an optimum cutting temperature (OCT) gel-sucrose mixture frozen by immersion in an isopentane (BDH Laboratory Supplies, UK) bath on dry ice and stored at -80°C until processing.

Cryosectioning of tissue: Frozen, coronal sections (spinal cord: 20 µm, brain: 30 µm) were cut with a Hacker-Bright cryostat (Hacker Instruments and Industries Inc., Winnsboro, SC) and mounted on charged glass slides (Superfrost Plus; Fisher Scientific, Pittsburgh, PA) so that adjacent CNS sections on each slide represented regions 100 µm and 150 µm apart for spinal cord and brain, respectively ((Tabata, Wilson et al. 2008)). The slides were stored at -80°C until histological processing. To ensure CNS sections were matched between animals, anatomical landmarks were verified using a mouse brain atlas (Paxinos and Franklin 2001).

### EXAMPLE 5

### HISTOLOGY

Histology and quantification of motor neurons: Spinal cord and brain sections from each animal were stained for Nissl body substance. At room temperature (RT), sections were rinsed in a graded ethanol series (95%: 15 min; 70%: 1 min; 50%: 1 min) to rehydrate and remove lipids and fixation chemicals from the tissue. The tissue were then rinsed for 2 min in two exchanges of distilled water, stained with twice-filtered cresyl violet stain (1.25 g cresyl violet acetate, 0.75 mL glacial acetic acid to 250 mL distilled water), rinsed in distilled water (1 minute), and then dehydrated (50% ethanol: 1 minute; 1% glacial acetic acid-70% ethanol: 2 minutes; 95% ethanol: 2 minutes; 95% ethanol: 3 dips; 100% ethanol: 1 minute) and cleared in xylene. The slides were then cover-slipped in Entellen mountant (Merck, Darmstadt, Germany). The tissue mounted slides were selected with a randomized start and sampled starting from the lumbar 2 region to the 6^{th}. The superior-inferior boundaries of the lumbar and thoracic spinal cord were initially determined by a ventral and dorsal root count and verified by comparison to a stereotaxic mouse atlas (Sidman, Angevine et al. 1971). The boundaries for the ventral horn was determined by the margins of the gray matter for the lateral, medial and ventral boundaries as well as with the aid of an artificial line drawn laterally through the central canal. Both the left and right ventral horns from 12 sections from each animal were counted. Alpha-motor neurons and γ-motor neurons with and without apoptotic structural changes were counted using strict morphological and size criteria. Non-apoptotic α-neurons were considered as the following: Large, multipolar cells with a round, pale nucleus; visible nucleolus; globular Nissl body staining of the cytoplasm; and a diameter of 30 - 45 µm. In doing so, astrocytes, oligodendrocytes and microglia were excluded from the "α-motor neuron" counts (Martin, 1999; Sepkuty et al., 2002; Tabata et al., 2008). Pyknotic or karyopyknotic α-neurons undergoing apoptosis were considered as those with a diameter of 30 µm and greater with a condensed and darkly stained nucleus, with or without vacuolization outside of the cell. Chromatolytic α-neurons undergoing apoptosis were considered as those with a diameter of 30 µm and greater with no discernable nucleus, with or without shifting of the nucleus from its central position to the periphery, disintegration of the Nissl bodies (chromophil substance), with or without vacuolization outside of the cell. Healthy γ-motor neurons were considered as those cells between 18 µm and 30 µm in size (Szumanska and Gadamski, 1977). Apoptotic γ-motor neurons were considered as those between 18 µm and 30 µm in size with a condensed and darkly stained nucleus with or without vacuolization outside of the cell. Those motor neurons with the nucleus (in the count for "healthy" α- and γ- motor neurons), nucleolus, and cytosol intact were included in the counts. Counts performed on non-treated animal sections served as controls. For results see FIGURE 5 and 8.

### EXAMPLE 6

### IMMUNOFLUORESCENCE

Tissue-mounted slides were processed for fluorescence microscopy as follows: Sections were rinsed in coplin jars for 45 minutes at room temperature (RT) in three changes of PBS. Sections were then incubated at RT for 30 minutes in a blocking permeabilization solution made of 5% normal goat serum (NGS), 5% normal rabbit serum (NRS) or 5% BlokHen II (Aves Labs, Inc., Tigard, OR), 0.05% Triton X-100 and PBS. Subsequently, the blocking solution was tipped off and the slides were rinsed in PBS for 2 minutes. Sections were then incubated overnight in a humidified chamber at 4°C with a PBS buffered solution that contained 1% NGS or 1% NRS, 1% bovine serum albumin (BSA) and one of the following primary antibodies: (a) rabbit anti-human/mouse ATF-3 (H-90) IgG (polyclonal, 1:500; Santa Cruz Biotechnology, Inc., Santa Cruz, CA), (b) anti-activated caspase-3 IgG (monoclonal, 1:250; Chemicon International, Temecula, CA) (see FIGURE 9), (c) goat antihuman/ mouse choline acetyl transferase (ChAT) IgG (polyclonal, 1:100; Chemicon International, Temecula, CA) (see FIGURE 6), (d) rabbit anti-mouse glial fibrillary acidic protein IgG (polyclonal, 1:250; GeneTex Inc., San Antonio, TX) (see FIGURE 7), (e) rabbit antihuman/ mouse ionized calcium binding adaptor molecule 1 (IBA-1) IgG (polyclonal, 1:500; Wako Pure Chemical Industries, Ltd, Richmond, VA), (f) mouse anti-human/mouse clone AT8 PHF-1 Tau (monoclonal, 1:200; Pierce Biotechnology Inc., Rockford, IL), (g) rabbit anti-human/mouse TAR DNA-binding protein 43 (TDP43) (1:100; ProteinTech Group Inc., Chicago, IL) (See FIGURE 14), (h) chicken anti-human/mouse β-tubulin 3 (TuJ1 antigen) (polyclonal, 1:100, Aves Labs, Inc., Tigard, OR), (i) rabbit anti-mouse tyrosine hydroxylase (polyclonal, 1:500, Chemicon International, Temecula, CA or polyclonal 1:1000, Affinity BioReagents, Golden, CO) (FIGURE 10), (j) rabbit anti human/mouse anti-pJunₛₑᵣ₇₃ IgG (polyclonal, 1:200; Chemicon International, Temecula, CA), (k) rabbit anti human/mouse anti-HSP-70 IgG (polyclonal, 1:200; Chemicon International, Temecula, CA), or (1) rabbit anti human/mouse anti-HSP-25 IgG (polyclonal, 1:100; Chemicon International, Temecula, CA). In each experiment, additional slides were incubated with the primary antibody and the secondary antibody omitted or with secondary antibody and primary antibody omitted to test for nonspecific staining. Where appropriate, positive control slides (Alzheimer's disease patient entorhinal cortex, obtained from Drs. Claudia Schwab and Patrick McGeer of the Kinsmen Laboratory, University of British Columbia, Vancouver, BC, Canada) were also processed as above for comparison. Following overnight incubation with the primary antibody, slides were rinsed for 45 minutes at RT in three changes of PBS. Immediately after, the slides were incubated in black chambers that excluded light for 1 hour at RT with the secondary antibody solutions containing 5% NGS or 5% NRS and fluorophore conjugated antibodies: AlexaFluor 568 goat anti-rabbit or rabbit anti-goat IgG (monoclonal, 1:250, absorption: 578 nm, emission 603 nm; Molecular Probes, Eugene, OR), AlexaFluor 546 goat antirabbit or rabbit anti-goat IgG (monoclonal; 1:250, absorption 556 nm, emission 573 nm; Molecular Probes, Eugene, OR) or fluorescein isothiocyanate (FITC) goat anti-rabbit IgG (polyclonal, 1:500, absorption: 494 nm, emission: 518 nm; Sigma, Saint Louis, MO). The secondary antibody solution was tipped off and slides were rinsed for 15 minutes in three changes of PBS. Tissue sections were cover-slipped with a 4'6 diamidino-2-phenylindole (DAPI) fluorescent mounting media (Vectashield, absorption: 360 nm, emission: 460 nm, Vector Laboratories, Burlington, ON, Canada) in order to label the nuclei and stored at 4°C in dark slide boxes to retard bleaching of the fluorescent labels.

### EXAMPLE 7

### TAU IMMUNOHISTOCHEMISTRY

Slide-mounted lumbar and thoracic spinal cord and brain sections were first rinsed for 45 minutes in 3 changes of PBS then quenched in 3% H₂O₂ in PBST (PBS + 0.5% Triton X-100) for 10 minutes. The slides were rinsed for 4 minutes in 2 changes of PBST. The MOM mouse Ig blocking reagent was prepared as per the manufacturer's instructions. Two drops of mouse Ig stock solution was added to 2.5 mL of PBS. The sections were incubated with this solution for 1 hour at RT. Following this step, sections were rinsed for 4 minutes in 2 changes of PBST and incubated (5 minutes) with the MOM diluent solution prepared by adding 600 V1 of MOM protein concentrate to 7.5 mL of PBS. The diluent was tipped off the slides and replaced by a solution comprised of primary phospho-Tau antibody diluted at 1:100 in the diluent solution prepared in the same manner as discussed above. The sections were incubated for 60 minutes at RT. Subsequently, sections were rinsed for 4 minutes in 2 changes of PBST and incubated for 10 minutes in the MOM biotinylated anti-mouse IgG reagent (10 V1 of MOM biotinylated anti-mouse IgG diluted in 2.5 mL of the MOM diluents). The sections were then rinsed in PBST and incubated (1 hour at RT) with the secondary antibody prepared by combining one drop of secondary antibody (biotinylated goat-anti-rabbit IgG) from the Vectastain ABC Elite kit (Vector Laboratories, Burlingame, CA, USA) was combined with 3 drops of serum stock to 10 mL of PBST. At 30 minutes after start of slide incubation with the secondary antibody, an immunoperoxidase solution was prepared by adding 2 drops of reagent A and 2 drops of reagent B (Vectastain Elite ABC kit, Vector Laboratories, Burlingame, CA, USA) to 2.5 mL of PBST. After one hour incubation of the slides with the secondary antibody solution, the slides were rinsed for 4 minutes in two changes of PBST and incubated for 30 minutes in the working immunoperoxidase solution. Next, the sections were rinsed for 4 minutes in 2 changes of PBST. During the rinses, the DAB liquid substrate was prepared for the next step. As per manufacturer's instructions, 2 drops of buffer stock solution, 5 mL of ddH₂O, 4 drops of DAB stock solution, and 2 drops of hydrogen peroxide solution were combined. The slides were incubated for 3 minutes at RT with this DAB solution and monitored for color development. The sections were rinsed for 5 minutes in ddH₂O to stop the reaction and rid slides of DAB substrate. A methyl green nuclear counterstain was used to elucidate nuclei and motor neuron somata. The 0.5% methyl green counterstain was prepared in the following manner: First, the sodium acetate buffer was prepared with 1.36 g of sodium acetate trihydrate to 100 mL of distilled water adjusted to pH 4.2 using glacial acetic acid. For every 100 mL of buffer, 0.5 g of methyl green (ethyl violet, Sigma-Aldrich, Oakville, ON, Canada) was added to make a 0.5% methyl green solution. Slide-mounted sections were stained in the above methyl green solution for 5 minutes at RT. Sections were rinsed in distilled water, until the water became clear. Next, sections were dehydrated quickly with 95% ethanol (5 dips) and 2 changes of 100% ethanol (5 dips in each change), then cleared by 10 dips of xylene. Finally, the slides were dried under a fume hood and cover-slipped with a resinous mounting media (Entellen, Merck, Darmstadt, Germany). No phosphorylated Tau aggregates were observed in the non-treated mice. Phosphorylated Tau aggregates were observed in the SG-fed mice (see FIGURE 13).

### EXAMPLE 8

### TYROSINE HYDROXYLASE IMMUNOHISTOCHEMISTRY

Labelling of dopaminergic neurons in brain sections was identified by antibodies for tyrosine hydroxylase (Morrow et al., 2001) and indirect immunoperoxidase staining. Sections were incubated in blocking serum for 2 hours then with the primary antibody (rabbit anti-tyrosine hydroxylase, 1:500, Affinity Bioreagents, Golden, CO, USA) for 3 hours at RT. Sections were rinsed and incubated in biotinylated goat anti-rabbit IgG (1:100; Vector laboratories Inc., Burlingame, USA) for 30 minutes at RT, then visualized using the ABC method (Vectastain Elite ABC kit, Vector Laboratories Inc.). See FIGURE 10.

### EXAMPLE 9

### CYTOCHROME C OXIDASE HISTOCHEMISTRY

Tissue-mounted slides were processed for fluorescence immunohistochemistry in a manner adapted from Seligman et al. (1968) and Wong-Riley (1979). Sections were rinsed in coplin jars for 15 minutes at RT in three changes of PBS and subsequently incubated with a media comprised of 50 mg of 3,3'-diaminobenzidine tetrahydrochloride (DAB) (DAB substrate system, Vector Laboratories, Burlingame, CA, USA), 90 mL 0.1M phosphate buffer, 20 mg cytochrome c (type III, Sigma-Aldrich Co., Oakville, ON, Canada), and 4 g of sucrose in a dish submerged in a water bath maintained at 37°C in the dark for 2 hours. The sections were monitored closely for the brown reaction product to appear within the tissue. Once a medium-brown color was achieved, slides were removed from the incubation media and rinsed for 15 minutes in three changes of PBS to stop the reaction. This was followed with a 2 minutes rinse in distilled water to remove residue from the slides. The slides were dried under a fume hood and cover-slipped with Entellen (Merck, Darmstadt, Germany). The results show no significant differences between the control mice and the SG-fed mice, although both groups showed a decline in cytochrome c oxidase over time, see FIGURE 11.

### EXAMPLE 10

### OIL RED O HISTOCHEMISTRY

Tissue-mounted slides were processed to evaluate lipid distribution with light microscopy with a protocol adapted from Dr. Roy Ellis (IMVS Division of Pathology, The Queen Elizabeth Hospital, Woodville, South Australia). First, an oil red O stock solution was prepared 2 hours prior to execution of the histochemical procedures. The oil red O stock stain solution was made by mixing oil red O (0.5 g, Sigma-Aldrich, Oakville, ON, Canada) and isopropanol (100 mL, Fisher Scientific, Nepean, ON, Canada) over a 40°C water bath. Immediately prior to beginning the oil red O histochemical procedures, the oil red O working solution was made by combining 20 mL of distilled water for every 30 mL of oil red O stock stain solution. This working solution was filtered five times to remove any oil red O that did not dissolve in the solvent. Slide-mounted sections were rinsed in coplin jars for 15 minutes at RT in three changes of PBS. Sections were then rinsed at RT with 60% isopropanol for 5 minutes. The spinal cord sections were then stained with a freshly prepared working solution of oil red O for 20 minutes and then rinsed with 60% isopropanol. Nuclear counterstaining was achieved by staining the sections with haematoxylin (haematoxylin, 7211, Richard Allan Scientific, Kalamazoo, MI, USA) for 1 minute. Finally, the sections were rinsed with distilled water, dried under a fume hood and cover-slipped with Entellen (Merck, Darmstadt, Germany). See FIGURE 12.

### EXAMPLE 11

### TISSUE VISUALIZATION AND IMAGE CAPTURE

Immunofluorescence protocol processed tissue sections were examined and captured using a Zeiss Axiovert 200M (Carl Zeiss Canada Limited, Toronto, ON). DAPI (blue fluorescence) visualization required a 359/461 nm absorption/emission filter. FITC required a 490,494/520 nm filter. Alexa Fluor 546™ (red fluorescence) utilized a 556,557/572,573 nm filter. During quantification at 40x magnification, two images were captured per lumbar and thoracic spinal cord section (i.e. left ventral horn and right ventral horn). Similarly, two images were captured per brain region and per dorsal or ventral root section. 40x images were 350 x 275 Vm in dimension. Images were captured using AxioVision 4.3 software. Data processed through immunofluorescence was analyzed using Zeiss Axiovert Zoom Axiovision 3.1 with AxioCam HRM. Non-fluorescent protocol processed tissue sections were examined and captured using the Motic B5 Professional Series 3.0 microscope (Motic Instruments Inc., Richmond, ON) and data were analyzed using Motic B5 Professional, Motic Images Advanced 3.0 (Motic Instruments Inc., Richmond, ON).

### EXAMPLE 12

### QUANTIFICATION OF IMMUNOREACTIVE CELL SOMATA OR INCLUSIONS

The numbers of immunoreactive somata or inclusions were counted across the ventral horn of the spinal cord, across brain hemispheres, or per dorsal or ventral root with the 40x objective lens in a 600 µm / 600 µm area within region perimeters defined by a mouse brain atlas (2001). For each animal, eight cross-sections per spinal cord or brain region were studied. An average of both the right and left ventral horn for each of the eight cross sections was generated giving a final averaged number for each animal in each of the spinal cord areas. For quantification of the level of mitochondrial activity (cytochrome c oxidase labelling) in the lumbar spinal cord or tyrosine hydroxylase immunoreactivity in the substantia nigra pars compacta and striatal sections, images captured using the Motic B5 Professional Series 3.0 microscope (Motic Instruments Inc., Richmond, ON) and analyzed using Motic B5 Professional, Motic Images Advanced 3.0 (Motic Instruments Inc., Richmond, ON) were scanned into a TIFF format file and analysed using the NIH Image J 1.37 software to measure density levels of antibody labelling. Eight to 12 sections from each mouse per region of interest were included.

### EXAMPLE 13

### STATISTICAL ANALYSES

Quantitative data obtained from each hemisphere of each spinal cord or brain section was averaged to generate a hemispherical average, then data averages for each animal was averaged prior to statistical analyses. Differences between the control and SG-fed mice were statistically evaluated using the Student's t-test with a p<0.05 being required for significance. All data are expressed as mean +/- standard error of the mean (SEM). Statistical significance was established using either an unpaired two-tailed *t*-test, one-way or two-way ANOVA followed by a Tukey's post-hoc or Fisher's LSD (GraphPad Prism v3.0, GraphPad Software Inc., San Diego, CA; Statistica for Windows v. 6.1 statistical software package, Statsoft, Tulsa, OK). Statistical analyses were reviewed independently by several reviewers from the Department of Statistics, University of British Columbia.

**TABLE 1**

| | Time-point 1 | Time-point 2 |
|---|---|---|
| **Mouse Strain, sex** | CD1 (outbred), male | |
| **Mouse age at study start** | 20 wk | |
| **Numbers (n)** | Control=14; SG-fed=16 | Control=7; SG-fed=8 |
| **SG Feeding Duration** | 15 wk | 15 wk |
| **Post-feeding survival duration** | 0 wk | 17 wk |
| **Mouse age at sacrifice** | 35 wk | 52 wk |
| | Leq extension | Leq extension* |
| | Rotarod | Rotarod |
| | Wirehanq | Wirehang* |
| | Velocity of movement | Velocity of movement |
| | Angular velocity | Angular velocity |
| | Percent time spent moving | Percent time spent moving |
| | Total distance moved (ns) | Total distance moved |
| | Total distance to zone border (ns) | Total distance to zone border* |
| **Behavioral Tests** | Total turn angle (ns) | Total turn angle |
| | Forelimb braking (ns) | Forelimb braking* |
| | Forelimb propulsion (ns) | Forelimb propulsion* |
| | Forelimb stance width (ns) | Forelimb stance width |
| | Forelimb stride length (ns) | Forelimb stride length |
| | Hind limb braking (ns) | Hind limb braking* |
| | Hind limb propulsion (ns) | Hind limb propulsion* |
| | Hind limb stance width (ns) | Hind limb stance width |
| | Hind limb stride length (ns) | Hind limb stride length |
| | Hind limb stride frequency (ns) | Hind limb stride frequency* |
| **Motor Neurons per lumbar cord section** | Healthy α-MN (ns) | Healthy α-MN: -55%* |
| | Chromatolytic α-MN (ns) | Chromatolytic α-MN (ns) |
| | Pyknotic α-MN (ns) | Pyknotic α-MN (ns) |
| | Total α-MN: -29%* | Total α-MN: -47* |
| | Total γ-MN (ns) | Total γ-MN (ns) |
| | Chromatolytic α-MN | |
| | (control group & SG-group independently over time) (ns) | |
| | Pyknotic α-MN | |
| | (control group and SG-group independently over time) (ns) | |
| | Total α-MN (control grouρ over time (ns) | |
| | Total α-MN (SG-fed group over time): -36%* | |
| | Total γ-MN | |
| | (control group and SG-group independently over time) (ns) | |
| **Cholinergic cell number** Choline acetyl transferase | Cholinergic neurons (-18%) | Cholinergic neurons (-32%) |
| | Total cholinergic cells (control group over time) (ns) | |
| | Total cholinergic cells (SG-fed group over time): -32%* | |
| **Stress induction** Heat shock protein-70 Activating transcription factor-3 Phosphorylated Junₛₑᵣ73 | Anti-Hsp-70: +1036%*** (VHLsc) | Anti-Hsp-70: +425%* (VHLsc) |
| | | Anti-Hsp-70: (ns, p=0.2793) (VHTsc) |
| | Anti-Hsp-70: (ns, p=0.9697) (VHTsc) | |
| | Anti-Hsp-70 Lsc (control group over time) (ns, p=0.7334) | |
| | Anti-Hsp-70 Lsc (SG-fed group over time) (ns, p=0.1223) | |
| | Anti-Hsp-70 Tsc (control group over time) (ns, p=0.2967) | |
| | Anti-Hsp-70 Tsc (SG-fed group over time) (ns, p=0.8319) | |
| | Anti-ATF-3: +242%*** | Anti-ATF-3: +101 %** |
| | Anti-ATF-3 immunoreactive cells (control group over time) (ns) | |
| | Anti-ATF-3 immunoreactive cells (SG-fed group over time): - 48%*** | |
| | Anti-pJun: +199%* | Anti-pJun (ns) |
| | Anti-pJun immunoreactive cells (control group over time) (ns) | |
| | Anti-pJun immunoreactive cells (SG-fed grouρ over time) (ns) | |
| **Apoptotic activity** Active Caspase-3 | Anti-active caspase 3: +78%*** | (ns) |
| | Apoptotic cells (control grouρ over time) (ns) | |
| | Apoptotic cells (SG-fed group over time) (ns) | |
| **Astrocyte proliferation** Glial fibrillary acidic protein | Anti-GFAP: +337%*** | Anti-GFAP: +119%* |
| | Astrocytes (control group over time): +41%* | |
| | Astrocytes (SG-fed group over time): -30%* | |
| **Microglia proliferation** Ionized calcium-binding adaptor molecule-1 | Anti-IBA1: +33%** | Anti-IBA1: +59%* |
| | Anti-IBA-1 immunoreactive cells (control group over time) (ns) | |
| | Anti-IBA-1 immunoreactive cells (SG-fed group over time) (ns) | |
| **Cytochrome c oxidase Activity** | (ns) | (ns) |
| | Optical density (control grouρ over time): -47%*** | |
| | Optical density (SG-fed group over time): -36%** | |
| **Dopaminergic cells** Tyrosine Hydroxylase | -34%*** | -39%* |
| | Optical density (control group over time) (ns) | |
| | Optical density (SG-group over time): -22%* | |
| **Lipid deposits** Oil red O | Lipid deposits Lsc VH: +28%* | Lipids deposits Lsc VH: +10%* |
| | Control Lsc over time: +29%* | |
| | SG-fed Lsc over time: ns, p=0.4864 | |
| | Control DR over time: ns, p=0.4864 | |
| | SG-fed DR over time: ns, p=0.8062 | |
| | Control VR over time: ns, p=0.1301 | |
| | SG-fed VR over time: ns, p=0.5478 | |
| **Phosphorylated tau aggregates** | - | + (in 2/8 of SG-fed group) |
| **Phosphorylated TDP-43 aggregates** | - | + (in 2/8 of SG-fed group) |

| | | |
|---|---|---|
| *p<0.05; **p<0.01; ***p<0.00 not significant = ns; Lsc = lumbar spinal cord; Tsc = thoracic spinal cord; ventral horn = VH; dorsal root=DR: - = not present; + = present | | |

While certain embodiments of the present invention have been described and/or exemplified above, it is contemplated that considerable variation and modification thereof are possible. Accordingly, the present invention is not limited to the particular embodiments described and/or exemplified herein.
Aarsland, D., Andersen, K., Larsen, J. P., Lolk, A., and Kragh-Sorensen, P. (2003) Prevalence and characteristics of dementia in Parkinson disease: an 8- year prospective study. Arch Neurol. 60, 387-92.
Arasaki, K. and Tamaki, M. (1998) A loss of functional spinal alpha motor neurons in amyotrophic lateral sclerosis. Neurology. 51, 603-5.
Arima, K., Hirai, S., Sunohara, N., Aoto, K., Izumiyama, Y., Ueda, K., Ikeda, K., and Kawai, M. (1999) Cellular co-localization of phosphorylated tau- and NACP/alpha-synuclein-epitopes in lewy bodies in sporadic Parkinson's disease and in dementia with Lewy bodies. Brain Res. 843, 53-61.
Barneoud, P. and Curet, O. (1999) Beneficial effects of lysine acetylsalicylate, a soluble salt of aspirin, on motor performance in a transgenic model of amyotrophic lateral sclerosis. Exp Neurol. 155, 243- 51.
Calne, D. B. and Eisen, A. (1989) The relationship between Alzheimer's disease, Parkinson's disease and motor neuron disease. Can J Neurol Sci. 16, 547-50.
Carter, R. J., Lione, L. A., Humby, T., Mangiarini, L., Mahal, A., Bates, G. P., Dunnett, S. B., and Morton, A. J. (1999) Characterization of progressive motor deficits in mice transgenic for the human Huntington's disease mutation. J Neurosci. 19, 3248-57.
Chapillon, P., Lalonde, R., Jones, N., and Caston, J. (1998) Early development of synchronized walking on the rotorod in rats. Effects of training and handling. Behav Brain Res. 93, 77-81.
Chen, C., Kano, M., Abeliovich, A., Chen, L., Bao, S., Kim, J. J., Hashimoto, K., Thompson, R. F., and Tonegawa, S. (1995) Impaired motor coordination correlates with persistent multiple climbing fiber innervation in PKC gamma mutant mice. Cell. 83, 1233-42.
Crawley, J. N. (1999) Behavioral phenotyping of transgenic and knockout mice: experimental design and evaluation of general health, sensory functions, motor abilities, and specific behavioral tests. Brain Res. 835, 18-26.
Crawley, J. N. (2000) What's Wrong With My Mouse? : Behavioral Phenotyping of Trangenic and Knockout Mice. 65--69.
Dauer, W. and Przedborski, S. (2003) Parkinson's disease: mechanisms and models. Neuron. 39, 889-909.
Dobson, C. M. (2003) Protein folding and misfolding. Nature. 426, 884-90.
Gerlai, R. and Roder, J. (1996) Spatial and nonspatial learning in mice: effects of S100 beta overexpression and age. Neurobiol Learn Mem. 66, 143-54.
Jones, B. J. and Roberts, D. J. (1968) A rotarod suitable for quantitative measurements of motor incoordination in naive mice. Naunyn Schmiedebergs Arch Exp Pathol Pharmakol. 259, 211.
Kokubo, Y., Kuzuhara, S., and Narita, Y. (2000) Geographical distribution of amyotrophic lateral sclerosis with neurofibrillary tangles in the Kii Peninsula of Japan. J Neurol. 247, 850-2.
Kurland, L. T. (1988a) Amyotrophic lateral sclerosis and Parkinson's disease complex on Guam linked to an environmental neurotoxin. Trends Neurosci. 11,51-4.
Kurland, L. T., Radhakrishnan, K., Williams, D. B., and Waring, S. C.(1994) Amyotrophic lateral sclerosis-parkinsonism-dementia complex on Guam: epidemiologic and etiological perspectives. 109-131.
Leenders, K. L., Salmon, E. P., Tyrrell, P., Perani, D., Brooks, D. J., Sager, H., Jones, T., Marsden, C. D., and Frackowiak, R. S. (1990) The nigrostriatal dopaminergic system assessed in vivo by positron emission tomography in healthy volunteer subjects and patients with Parkinson's disease. Arch Neurol. 47, 1290-8.
Lucking, C. B. and Brice, A. (2000) Alpha-synuclein and Parkinson's 204 disease. Cell Mol Life Sci. 57,1894-908.
Martin, L. J. (1999) Neuronal death in amyotrophic lateral sclerosis is apoptosis: possible contribution of a programmed cell death mechanism. J Neuropathol Exp Neurol. 58, 459-71.
Muchowski, P. J. and Wacker, J. L. (2005) Modulation of neurodegeneration by molecular chaperones. Nat Rev Neurosci. 6, 11- 22.
Norflus, F., Tifft, C. J., McDonald, M. P., Goldstein, G., Crawley, J. N., Hoffmann, A., Sandhoff, K., Suzuki, K., and Proia, R. L. (1998) Bone marrow transplantation prolongs life span and ameliorates neurologic manifestations in Sandhoff disease mice. J Clin Invest. 101, 1881-8.
Odle, T. G. (2003) Alzheimer disease and other dementias. Radiol Technol. 75, 111-35; quiz 136-9.
Rowland, L. P. and Shneider, N. A. (2001) Amyotrophic lateral sclerosis. N Engl J Med. 344, 1688-700.
Sango, K., McDonald, M. P., Crawley, J. N., Mack, M. L., Tifft, C. J., Skop, E., Starr, C. M., Hoffmann, A., Sandhoff, K., Suzuki, K., and Proia, R. L.(1996) Mice lacking both subunits of lysosomal beta-hexosaminidase display gangliosidosis and mucopolysaccharidosis. Nat Genet. 14, 348- 52.
Sango, K., Yamanaka, S., Hoffmann, A., Okuda, Y., Grinberg, A., Westphal, H., McDonald, M. P., Crawley, J. N., Sandhoff, K., Suzuki, K., and et, a. l. (1995) Mouse models of Tay-Sachs and Sandhoff diseases differ in neurologic phenotype and ganglioside metabolism. Nat Genet. 11, 170-6.
Schapira, A. H. and Olanow, C. W. (2003) Rationale for the use of dopamine agonists as neuroprotective agents in Parkinson's disease. Ann Neurol. 53 Suppl 3, S149-57; discussion S 157-9.
Sepkuty, J. P., Cohen, A. S., Eccles, C., Rafiq, A., Behar, K., Ganel, R., Coulter, D. A., and Rothstein, J. D. (2002) A neuronal glutamate transporter contributes to neurotransmitter GABA synthesis and epilepsy. J Neurosci. 22, 6372-9.
Steele, J. C. and Guzman, T. (1987) Observations about amyotrophic lateral sclerosis and the parkinsonism- dementia complex of Guam with regard to epidemiology and etiology. Can J Neurol Sci. 14, 358-62.
Szumanska, G. and Gadamski, R. (1977) [Histochemical study of rat brain following intoxication with dichlorvos (DDVP)]. Neuropatol Pol. 15, 523- 35.
Tabata, R. C., Wilson, J. M., Ly, P., Zwiegers, P., Kwok, D., Van Kampen, J. M., Cashman, N., and Shaw, C. A. (2008) Chronic Exposure to Dietary Sterol Glucosides is Neurotoxic to Motor Neurons and Induces an ALSPDC Phenotype. Neuromolecular Med. 10, 24-39.
Vaphiades, M. S., Husain, M., Juhasz, K., and Schmidley, J. W. (2002) Motor neuron disease presenting with slow saccades and dementia. Amyotroph Lateral Scler Other Motor Neuron Disord. 3, 159-62.
Verghese, J., Lipton, R. B., Hall, C. B., Kuslansky, G., Katz, M. J., and Buschke, H. (2002) Abnormality of gait as a predictor of non-Alzheimer's dementia. N Engl J Med. 347, 1761-8.
Whitfield,D. M., and Douglas, S. P. (1996) Glycosylation reactions - present status future directions. Glycoconjugate Journal 13, 5-17.
Whitehouse, P. J., Price, D. L., Struble, R. G., Clark, A. W., Coyle, J. T., and Delon, M. R. (1982) Alzheimer's disease and senile dementia: loss of neurons in the basal forebrain. Science. 215, 1237-9.
Wilson, J. M., Khabazian, I., Pow, D. V., Craig, U. K., and Shaw, C. A. (2003d) Decrease in glial glutamate transporter variants and excitatory amino acid receptor down-regulation in a murine model of ALS-PDC. Neuromolecular Med. 3, 105-18.
Wilson, J. M., Khabazian, I., Wong, M. C., Seyedalikhani, A., Bains, J. S., Pasqualotto, B. A., Williams, D. E., Andersen, R. J., Simpson, R. J., Smith, R., Craig, U. K., Kurland, L. T., and Shaw, C. A. (2002a) Behavioral and neurological correlates of ALS-parkinsonism dementia complex in adult mice fed washed cycad flour. Neuromolecular Med. 1,207-21.
Wilson, J. M., Khabazian, I., Wong, M. C., Seyedalikhani, A., Bains, J. S., Pasqualotto, B. A., Williams, D. E., Andersen, R. J., Simpson, R. J., Smith, R., Craig, U. K., Kurland, L. T., and Shaw, C. A. (2002b) Behavioral and neurological correlates of ALS-parkinsonism dementia complex in adult mice fed washed cycad flour. Neuromolecular Med. 1,207-21.
Wilson, J. M., Petrik, M. S., Grant, S. C., Blackband, S. J., Lai, J., and Shaw, C. A. (2004c) Quantitative measurement of neurodegeneration in an ALS-PDC model using MR microscopy. Neuroimage. 23, 336-43.
Wilson, J. M., Petrik, M. S., Moghadasian, M. H., Grant, S. C., Blackband, S. J., Krieger, C., Craig, U. K., and Shaw, C. A. (2005) The Progression of Motor Deficits and CNS Pathology in ALS-PDC. Soc Neurosci. Abstr. online.
Yokoyama, K., Ikebe, S., Komatsuzaki, Y., Takanashi, M., Mori, H., Mochizuki, H., and Mizuno, Y. (2002) [A 68-year-old woman with dementia and parkinsonism]. No To Shinkei. 54, 175-84.
Zoccolella, S., Palagano, G., Fraddosio, A., Russo, I., Ferrannini, E., Serlenga, L., Maggio, F., Lamberti, S., and Iliceto, G. (2002) ALS-plus: 5 cases of concomitant amyotrophic lateral sclerosis and parkinsonism. Neurol Sci. 23 Suppl 2, S123-4.

## Claims

1. An animal model of a neurodegenerative disease comprising a non-human animal that has been administered with a neurotoxic compound wherein the neurotoxic compound is synthetic and/or purified stigmasterol glucoside, and the non-human animal is a rodent.

2. A method of monitoring a neurodegenerative disease in a non-human animal, the method comprising the steps of administering to the non-human animal a composition consisting essentially of a neurotoxic sterol glucoside; and
monitoring the neurodegenerative disease in the non-human animal by using at least one behavioral abnormality test or at least one post-mortem test for neurodegeneration;
wherein the neurotoxic sterol glucoside is synthetic and/or purified stigmasterol glucoside and the non-human animal is a rodent.

3. The model or method of claim 1 or claim 2 wherein the non-human animal is a mouse.

4. The method of claim 2 wherein the step of administering to the non-human animal the composition comprising the neurotoxic sterol glucoside comprises the steps of modifying the non-human animal's daily diet by adding the composition comprising the neurotoxic sterol glucoside to the diet and feeding the diet to the non-human animal.

5. The method of claim 2 wherein the step of administering to the non-human animal the composition comprising the neurotoxic sterol glucoside comprises the step of administering the composition comprising the neurotoxic sterol glucoside to the non-human animal by gavage feeding.

6. The method of any one of claims 2, 4 and 5 wherein the neurodegenerative disease is monitored by a test selected from a leg extension test, a gait length test, a rotarod test, a wire hang test, a water maze test, a radial arm maze test, a Digigate system test, an Ethovision test, an anxiety test, a depression test, and an olfactory system test.

7. The model or method of any one of the preceding claims wherein the neurodegenerative disease is selected from neuropathy, peripheral neuropathy, Pick's disease, amyotrophic lateral sclerosis, early onset Parkinson's disease, late onset Parkinson's disease, and Alzheimer's disease.

8. The model or method of any one of the preceding claims wherein the neurodegenerative disease is selected from amyotrophic lateral sclerosis, early onset Parkinson's disease, late onset Parkinson's disease, and Alzheimer's disease.

## Patentansprüche

1. Tiermodell für eine neurodegenerative Erkrankung, umfassend ein Tier, dem eine neurotoxische Verbindung verabreicht wurde, wobei die neurotoxische Verbindung synthetisches und/oder gereinigtes Stigmasteringlucosid ist und das Tier ein Nagetier ist.

2. Verfahren zum Verfolgen einer neurodegenerativen Erkrankung bei einem Tier, wobei das Verfahren die Schritte umfasst: Verabreichen einer Zusammensetzung, die im Wesentlichen aus einem neurotoxischen Steringlucosid besteht, an ein Tier; und
Verfolgen der neurodegenerativen Erkrankung bei dem Tier unter Verwendung wenigstens eines Tests auf Verhaltensanomalie oder wenigstens eines Post-Mortem-Tests für Neurodegeneration;
wobei das neurotoxische Steringlucosid synthetisches und/oder gereinigtes Stigmasteringlucosid ist und das Tier ein Nagetier ist.

3. Modell oder Verfahren nach Anspruch 1 oder Anspruch 2, wobei das Tier eine Maus ist.

4. Verfahren nach Anspruch 2, wobei der Schritt des Verabreichens der das neurotoxische Steringlucosid umfassenden Zusammensetzung an das Tier die Schritte Modifizieren der täglichen Nahrung des Tiers durch Zugeben der das neurotoxische Steringlucosid umfassenden Zusammensetzung zu der Nahrung und Füttern des Tiers mit der Nahrung umfasst.

5. Verfahren nach Anspruch 2, wobei der Schritt des Verabreichens der das neurotoxische Steringlucosid umfassenden Zusammensetzung an das Tier den Schritt des Verabreichens der das neurotoxische Steringlucosid umfassenden Zusammensetzung an das Tier durch Sondenfütterung umfasst.

6. Verfahren nach einem der Ansprüche 2, 4 und 5, wobei die neurodegenerative Erkrankung durch einen Test verfolgt wird, der ausgewählt ist aus einem Leg-Extension-Test, einem Schrittlängentest, einem Rotarod-Test, einem Wire-Hang-Test, einem Wasserlabyrinth-Test, einem Radialarmlaybyrinth-Test, einem Digigatesystem-Test, einem Ethovision-Test, einem Angst-Test, einem Depressionstest und einem Test des olfaktorischen Systems.

7. Modell oder Verfahren nach einem der vorhergehenden Ansprüche, wobei die neurodegenerative Erkrankung ausgewählt ist aus Neuropathie, peripherer Neuropathie, Pick-Krankheit, amyotropher Lateralsklerose, Early-Onset-Parkinson-Krankheit, Late-Onset-Parkinson-Krankheit und Alzheimer-Krankheit.

8. Modell oder Verfahren nach einem der vorhergehenden Ansprüche, wobei die neurodegenerative Erkrankung ausgewählt ist aus amyotropher Lateralsklerose, Early-Onset-Parkinson-Krankheit, Late-Onset-Parkinson-Krankheit und Alzheimer-Krankheit.

## Revendications

1. Modèle animal d'une maladie neurodégénérative comprenant un animal non humain auquel a été administré un composé neurotoxique où le composé neurotoxique est un glucoside de stigmastérol synthétique et/ou purifié, et l'animal non humain est un rongeur.

2. Procédé de surveillance d'une maladie neurodégénérative chez un animal non humain, le procédé comprenant les étapes d'administration à l'animal non humain d'une composition consistant essentiellement en un glucoside de stérol neurotoxique; et
de surveillance de la maladie neurodégénérative chez l'animal non humain au moyen d'au moins un test d'anomalie comportementale ou d'au moins un test post-mortem pour la neurodégénérescence;
où le glucoside de stérol neurotoxique est un glucoside de stigmastérol synthétique et/ou purifié et l'animal non humain est un rongeur.

3. Modèle ou procédé selon la revendication 1 ou la revendication 2 où l'animal non humain est une souris.

4. Procédé selon la revendication 2 où l'étape d'administration à l'animal non humain de la composition comprenant le glucoside de stérol neurotoxique comprend les étapes de modification du régime journalier de l'animal non humain par addition de la composition comprenant le glucoside de stérol neurotoxique au régime et d'alimentation de l'animal non humain avec le régime.

5. Procédé selon la revendication 2 où l'étape d'administration à l'animal non humain de la composition comprenant le glucoside de stérol neurotoxique comprend l'étape d'administration de la composition comprenant le glucoside de stérol neurotoxique à l'animal non humain par alimentation par gavage.

6. Procédé selon l'une quelconque des revendications 2, 4 et 5 où la maladie neurodégénérative est surveillée par un test choisi parmi un test d'allongement des pattes, un test de longueur de la démarche, un test Rotarod, un test wire hang, un test avec labyrinthe d'eau, un test avec labyrinthe à bras radiaux, un test avec système Digigate, un test Ethovision, un test d'anxiété, un test de dépression et un test du système olfactif.

7. Modèle ou procédé selon l'une quelconque des revendications précédentes où la maladie neurodégénérative est choisie parmi la neuropathie, la neuropathie périphérique, la maladie de Pick, la sclérose latérale amyotrophique, la maladie de Parkinson à déclenchement précoce, la maladie de Parkinson à déclenchement tardif et la maladie d'Alzheimer.

8. Modèle ou procédé selon l'une quelconque des revendications précédentes où la maladie neurodégénérative est choisie parmi la sclérose latérale amyotrophique, la maladie de Parkinson à déclenchement précoce, la maladie de Parkinson à déclenchement tardif et la maladie d'Alzheimer.
